# EUROPÄISCHE PATENTSCHRIFT

(11) **EP 2 316 375 B1**
(45) Veröffentlichungstag und Bekanntmachung des Hinweises auf die Patenterteilung: **27.01.2016**
(21) Anmeldenummer: 10014123.3
(22) Anmeldetag: 29.10.2010
(51) Int. Cl.: A61C 19/00, A61L 2/18

(54) **Pflegevorrichtung**
Cleaning device
Dispositif de soin

(30) Priorität: 02.11.2009 DE 102009051619; 02.11.2009 DE 102009051620
(43) Veröffentlichungstag der Anmeldung: 04.05.2011
(73) Patentinhaber: Firma SycoTec GmbH & Co. KG, 88299 Leutkirch im Allgäu (DE)
(72) Erfinder: Buchmann, Siegfried, 88319 Aitrach (DE); Abrell, Werner, 88317 Aichstetten (DE)
(74) Vertreter: Pfister, Stefan Helmut Ulrich

(56) Entgegenhaltungen:
- EP-A1- 1 779 770
- DE-A1- 4 235 699
- DE-T2- 69 107 437
- DE-U1- 9 110 108
- US-A- 5 057 283

## Beschreibung

Die Erfindung betrifft eine Pflegevorrichtung für wenigstens ein dentaltechnisches Gerät, welches zumindest ein Vorratsgefäß für ein insbesondere fluides Reinigungs- oder Pflegemedium, wenigstens eine Reinigungskammer zur Anordnung des dentaltechnischen Gerätes und wenigstens eine das Vorratsgefäß und die Reinigungskammer verbindende Medienleitung umfasst.

In der Europäischen Patentschrift 300 945 ist eine gattungsgemäße Pflegevorrichtung beschrieben.

Aufgabe gattungsgemäßer Pflegevorrichtungen ist es, eine ordnungsgemäße und insbesondere keimfreie Reinigung der dentaltechnischen Geräte einerseits, aber auch eine Wartung beziehungsweise Pflege der oftmals mit rotierenden Elementen ausgestatteten Geräte andererseits durchzuführen. Die Pflegevorrichtung hat daher grundsätzlich auch mehrere Aufgaben. Neben einer Reinigungsaufgabe sind Desinfektionsaufgaben oder auch Wartungs- beziehungsweise Schmieraufgaben durch die Pflegevorrichtung zu erfüllen.

Der Begriff "dentaltechnische Geräte" ist dabei sehr umfassend zu verstehen, hierunter werden zahnärztliche Handstücke, Werkzeuge oder auch Antriebselemente wie Turbinen und so weiter verstanden.

Üblicherweise werden die Reinigungs- beziehungsweise Pflegemedien, oftmals in flüssiger Phase, in je einem Vorratsgefäß vorgehalten und dann bei Bedarf, zum Beispiel mit Druckluftunterstützung oder mit Hilfe einer Pumpe und so weiter in die Reinigungskammer gefördert, wobei das dentaltechnische Gerät in der Reinigungskammer auf einem entsprechenden Zapfen oder Reinigungsplatz aufgesetzt ist und mit Hilfe der Pflegevorrichtung sowohl eine innerliche als auch eine äußere Reinigung und Pflege des Gerätes erfolgt. Unter einer inneren Pflege beziehungsweise Reinigung wird dabei verstanden, dass die im dentaltechnischen Gerät sowieso vorgesehenen Medienleitungen, zum Beispiel für Druckluft oder Wasser beziehungsweise Mundspülbeziehungsweise Desinfektionslösung, auch zur Leitung der Pflegemittel verwendet werden und dabei gleichzeitig durch die Reinigungsmedien gespült und dadurch gereinigt und desinfiziert werden.

Eine ordnungsgemäße und auch die entsprechenden Hygieneanforderungen erfüllende Reinigung beziehungsweise Pflege der dentaltechnischen Geräte kann natürlich nur dann erfolgen, wenn entsprechendes Reinigungs- oder Pflegemedium in dem Vorratsgefäß zur Verfügung steht. Bei den bekannten Geräten verläßt man sich auf eine optische Überprüfung des Füllstandes des Reinigungs- und Pflegemediums in dem Vorratsgefäß durch den Benutzer, zum Beispiel einer Zahnarzthelferin oder anderes Personal. Ist der Füllstand aber zu gering, so ist unter Umständen eine ordnungsgemäße Reinigung und Pflege des dentaltechnischen Gerätes, das heißt, beispielsweise eine ausreichend lange Reinigung beziehungsweise eine Reinigung mit einer ausreichenden Menge an Reinigungsmedium die Keimabtötung sicherstellt, nicht mehr möglich und die Hygieneanforderungen können nicht eingehalten werden.

Die DE 4235699 offenbart eine Vorrichtung zum Reinigen und/oder Desinfizieren und/oder Pflegen von ärztlichen oder zahnärztlichen Handstücken mit nicht näher definierten Füllstandsmessern.

Die EP 1779770 offenbart eine Reinigungsvorrichtung für Endoskope, wobei nur der Füllstand der die Endoskope beinhaltenden Kammer überwacht wird.

Ausgehend von diesem Stand der Technik ist es Aufgabe der vorliegenden Erfindung eine Pflegevorrichtung für dentaltechnische Geräte zur Verfügung zu stellen, die einen möglichst hohen Grad an Hygiene an dem gereinigten und gepflegten dentaltechnischen Gerät ermöglicht.

Zur Lösung dieser Aufgabe geht die Erfindung aus von einer Pflegevorrichtung, wie eingangs beschrieben, und schlägt vor, dass eine Messeinrichtung zur Ermittlung der im Vorratsgefäß vorhandenen

Menge des Mediums vorgesehen ist und die Meßeinrichtung wenigstens einen Drucksensor aufweist. Bei diesem Sensor handelt es sich insbesondere um einen datentechnisch auslesbaren Sensor, der beispielsweise über eine entsprechende Datenleitung mit einer Auswerte- und Recheneinheit verbunden werden kann, die die durch den Sensor erfassten Rohdaten umgehend umsetzt und für weitere Berechnungen, insbesondere für die Ermittlung der im Vorratsgefäß vorhandenen Menge und/oder den Füllstand des Mediums heranzieht. Hierbei ist vorgesehen, dass ein im Vorratsgefäß herrschender statischer Druck und/oder eine bei der Entnahme des Mediums auftretende Druckänderung erfassbar ist, um hieraus einen Füllstand im Vorratsgefäß beziehungsweise die dort vorhandene Menge an Medium oder aber eine Änderung des Füllstandes beziehungsweise der Medienmenge abzuleiten.

In einer Messanordnung, welche nicht Teil der beanspruchten Erfindung ist, ist z. B. eine kapazitive Messung vorgesehen, wobei der entsprechende Sensor beispielsweise in Form von Elektroden aufgebaut ist. Es wird hierbei dann der Füllstand aus einer Änderung der elektrischen Kapazität zwischen den Elektroden detektiert, wenn diese von wechselnden Füllständen des Mediums umgeben werden. Ist der Sensor als Schwimmsensor ausgebildet, so kann hier beispielsweise eine Zweipunktüberwachung mit einem Doppelschwimmerschalter durchgeführt werden. Hierdurch können Grenzwerte für den Füllstand detektiert werden, um hierüber dann eine entsprechende Steuerung der Medienentnahme durchführen zu können. Bei einer entsprechenden Ausführung des Schwimmsensors mit einem induktiven Sensor besteht auch die Möglichkeit, dass eine kontinuierliche Erfassung des Füllstandes durchgeführt wird, sofern die Auswerteinheit, die in einer bevorzugten Weiterbildung der Erfindung in der Pflegevorrichtung vorgesehen, über entsprechende Auswertemöglichkeiten verfügt. Im Allgemeinen ist die Auswerteinheit dazu geeignet, die durch den Sensor erfassten Werte für die Änderung der elektrischen Kapazität, der Schwimmerposition und/oder den statischen Druck beziehungsweise die Änderung des Druckes im Vorratsgefäß auszuwerten, um hierüber dann zum Einen einen Wert für den Füllstand auszugeben, der beispielsweise durch eine optische Anzeige komuniziert wird beziehungsweise um den Wert an eine Steuereinheit weiterzugeben, die dann beispielsweise die Entnahme des Mediums beziehungsweise dessen Entnahmedauer aus dem Vorratsgefäß steuert.

Durch den erfindungsgemäßen Vorschlag ist es nun möglich, den Füllstand an fluidischem Reinigungs- und Pflegemedium in dem Vorratsgefäß elektronisch auszuwerten. Durch die Erfindung eröffnet sich die Möglichkeit einer elektronischen Erkennung und Verarbeitung des Füllstandes mit einer Vielzahl von davon ableitbaren Optionen. Es ist zum Beispiel möglich, den aktuellen Füllstand auf einer Anzeige zu visualisieren und so dem Benutzer über den aktuellen Stand zu unterrichten. Es ist aber auch möglich, akustische und/oder optische Warnsignale auszugeben, wenn zum Beispiel ein Mindest- oder auch ein Maximalstand unter- beziehungsweise überschritten wird. Somit ist es zum Beispiel auch möglich, einen Überfüllschutz zu realisieren, da insbesondere ein zu hoher statischer Druck unter Umständen zu einem erhöhten Verbrauch von Medium führt.

Durch den erfindungsgemäßen Vorschlag wird die Möglichkeit eröffnet, eine zuverlässige Pflege der dentaltechnischen Geräte zu erreichen, da zum Beispiel bei einem zu niedrigen Medienfüllstand das Gerät blockiert und eine entsprechende, automatisch angestoßene Pflege- beziehungsweise Reinigung des dentaltechnischen Gerätes unterbleibt und zum Beispiel ein akustisches Warnsignal ertönt. Neben einer erheblichen Verbesserung der Gerätereinigung und damit der gesamten Hygiene des zahnärztlichen Bereiches wird aber auch eine Erhöhung der Standzeiten der eingesetzten dentaltechnischen Geräte erreicht, da zuverlässig eine vollständige Reinigung und Wartung, das heißt, Schmierung oder eine sonstige Wartung des dentaltechnischen Gerätes erfolgt. Das Risiko von falsch behandelten Geräten oder Instrumenten wird erheblich reduziert und ein frühzeitiger Funktionsausfall des Gerätes vermieden, was zu entsprechenden Einsparungen an Kosten führt.

Der Einsatzbereich der Erfindung erstreckt sich dabei auf fluidische Reinigungs- oder Pflegemedien. Hiervon umfasst sind fließfähige Reinigungs- oder Pflegemedien, wie zum Beispiel flüssige Reinigungs- oder Pflegemittel, gegebenenfalls aber auch gasförmige Reinigungs- oder Pflegemittel. Dem Begriff "Fluiden" oder "Flüssigkeit" gleichwertig ist in dieser Anmeldung der Begriff "Medium" gesetzt.

Der erfindungsgemäße Vorschlag eröffnet aber auch noch eine weitere Verbesserung!

Üblicherweise wird das Reinigungs- oder Pflegemedium durch eine Pumpe aus dem Vorratsgefäß abgesaugt und mit Hilfe einer Dosiervorrichtung und einer Medienleitung in die Reinigungskammer gefördert. Der Einsatz von Druckluft ist bei zahnärztlichen Geräten bekannt und wird zum Beispiel zur Trocknung oder als Reinigungsluft bei der Behandlung eingesetzt. Es sind auch mit Druckluft angetriebenen Turbinen als Rotationsantriebe bekannt. Der Einsatz einer Pumpe ist daher für die Förderung des Mediums zur Reinigungskammer nicht zwingend notwendig, diese Aufgabe kann durch die Druckluft erfolgen. Das Entnehmen von Reinigungs-/Pflegemedium aus dem Vorratsgefäß erfolgt dabei nun allein durch die Gewichtskraft, wobei der Förderdruck mit abnehmendem Füllstand geringer wird. Wird aber nun der Druck im Vorratsgefäß, insbesondere die Druckänderung überwacht, so kann über eine Einflussnahmen auf die Dosiervorrichtung sichergestellt werden, dass unabhängig von dem Füllstand im Vorratsgefäß immer eine gleichbleibende Entnahmemenge an Medium für den jeweiligen Pflege- beziehungsweise Reinigungsvorgang zur Verfügung steht.

Es ist klar, dass dieser Vorzug sowohl bei Vorratsgefäßen, die mit Reinigungsmittel, wie auch bei Vorratsgefäßen, die mit dem Pflegemittel oder Desinfektionsmittel befüllt sind, besteht.

So kombiniert der erfindungsgemäße Vorschlag eine verbesserte Hygiene beziehungsweise Pflegesicherheit der oftmals kostspieligen Geräte mit gleichbleibender Qualität der Reinigung beziehungsweise Pflege bei geringerem Aufwand, da Fördermittel wie Pumpen und so weiter, die das Medium aus dem Vorratsgefäß abziehen, eingespart werden.

In einer bevorzugten Variante der Erfindung wird vorgeschlagen, dass eine Auswerteinheit für den durch den Sensor erfassten statischen Druck beziehungsweise die Druckänderung vorgesehen ist. Die Auswerteinheit ist dabei zum Beispiel als Teil einer umfassenderen Gerätesteuerung realisiert. Die Auswerteinheit erhält die entsprechenden Sensorinformationen des Sensors und ermittelt hieraus einen Wert, der dem Füllstand oder dem Vorratsvolumen an noch in dem Vorratsgefäß vorhandenen (Reinigungs- oder Pflege-)Medium entspricht. Dabei ist es grundsätzlich möglich, dass auch mit entsprechenden Druckänderungen, zum Beispiel vor und nach Entnahme von Medium im Sinne der Erfindung verfahren wird. Das heißt, die gesamte Anordnung, insbesondere aber auch die Auswerteinheit wie auch die Sensoren sind alternativ auch so ausgerichtet, dass über die Drucksensoren auch eine Druckänderung auswertbar ist.

In einer weiteren bevorzugten Ausgestaltung der Erfindung ist vorgesehen, dass eine Steuereinheit für die Entnahmemenge beziehungsweise Entnahmedauer des Reinigungs- oder Pflegemediums aus dem Vorratsgefäß vorgesehen ist. Diese ist mit der Auswerteinheit verbunden und wirkt auf die Dosiervorrichtung, wobei die Steuerung der Entnahmemenge beziehungsweise Entnahmedauer anhand der aus den Sensorinformationen abgeleiteten Werte für den Füllstand beziehungsweise die Medienmenge erfolgt. Durch eine solche Ausgestaltung wird erreicht, dass, unabhängig von dem tatsächlichen Füllstand von Medium in dem Vorratsgefäß, letztendlich also auch unabhängig von dem in der Medienleitung zur Dosiervorrichtung herrschenden Druck, immer die gleiche Entnahmemenge an Medium für eine zuverlässige und vollständige Reinigung beziehungsweise Pflege und Wartung des dentaltechnischen Gerätes oder Instrumentes zur Verfügung steht. Dabei ist zu beachten, dass mit im Zuge der Füllstandsabsenkung abnehmendem statischen Druck die Fließgeschwindigkeit des Mediums durch die Medienleitung vom Vorratsgefäß zur Dosiervorrichtung absinkt und dann zum Beispiel durch eine entsprechende Erhöhung der Entnahmedauer wieder eine gleiche Entnahmemenge erreicht wird.

Sowohl die Auswerteinheit wie auch die Steuereinheit sind dabei zum Beispiel Teile einer Gerätesteuerung der gesamten Pflegevorrichtung und zum Beispiel als Software in einem Microcontroler oder ähnlichem untergebracht.

Geschickterweise ist eine optische oder akustische Anzeige vorgesehen, die mit der Auswerteinheit beziehungsweise der Steuereinheit verbunden ist. Hierüber ist dem Benutzer jederzeit signalisierbar, welchen Vorrat er an den verschiedenen Medien noch hat. Mit Hilfe der Anzeige sind natürlich auch entsprechende Fehlermeldungen (zu niedriger Stand, Überfüllung und so weiter) optisch und/oder akustisch signalisierbar.

In einer erfindungsgemäßen Variante ist vorgesehen, dass der Sensor am Boden, im Bereich eines Auslasses des Vorratsgefäßes, im Medium, an der Wand des Vorratsgefäßes und hierbei insbesondere an dessen Außenwand und/oder in der Medienleitung angeordnet ist. Des Weiteren ist alternativ hierzu vorgesehen, dass bei Verwendung eines Drucksensors, dieser über eine Druckleitung mit dem Vorratsgefäß, insbesondere im Bodenbereich des Vorratsgefäßes verbunden ist. Der Vorzug der Erfindung ist, dass die Anordnung des Sensors sehr variabel gestaltbar ist. Eine bevorzugte Variante, die einen Drucksensor aufweist, ist dadurch angegeben, dass eine Druckleitung zwischen dem Vorratsgefäß und dem Drucksensor vorgesehen ist. Der Drucksensor selber ist gemäß der Erfindung zum Beispiel darauf ausgerichtet, mit einem flüssigen Medium zusammenzuwirken. Es ist aber auch möglich, dass der Drucksensor den Gasdruck der zum Beispiel oberhalb der Druckleitung befindlichen Gassäule misst.

Die Anordnung des Sensors ist variabel, er ist zum Beispiel ortsfest angeordnet oder auch frei beweglich, zum Beispiel als Schwimmsensor realisierbar. Dabei ist der Sensor gemäß der Erfindung ausreichend empfindlich, um die auftretende Änderungen zu erfassen oder aber es wird eine entsprechende Differenzmessung mit Hilfe eines weiteren Sensors realisiert. Günstigerweise ist vorgesehen, dass bei Erfassung von Druckwerten eine kontinuierliche Erfassung des statischen Druckes beziehungsweise eine kontinuierliche Erfassung der Druckänderung erfolgt, wobei insbesondere die Druckänderung während der Entnahme des Mediums von Interesse ist, um so die Entnahmedauer zu beeinflussen.

Zwischen dem Vorratsgefäß und der Reinigungskammer ist günstigerweise eine Dosiervorrichtung für das Medium angeordnet. So werden zum Beispiel in einer zentralen Medienleitung, welche von der Dosiervorrichtung zur Reinigungskammer führt, normalerweise mehrere Medienzuleitungen von verschiedenen Vorratsgefäßen zusammengeführt, um so über die gleiche Medienleitung unterschiedliche Medien zum zu reinigenden, dentaltechnischen Gerät oder Instrument zu führen. Die Dosiervorrichtung ist gegebenenfalls auch komplexer ausgestaltet, um den verschiedenen Anforderungen gerecht zu werden. Dabei hat die Dosiervorrichtung auch die Aufgabe sicherzustellen, dass eine ausreichende Menge von Reinigungs- beziehungsweise Pflegemedium zur Verfügung gestellt wird, um eine vollständige und befriedigende Reinigung beziehungsweise Wartung sicherzustellen. Geschickterweise besitzt daher die Dosiervorrichtung mindestens ein Ventil, zum Beispiel ein elektromagnetisch ansteuerbares Ventil.

Gegebenenfalls ist zusätzlich eine weitere Medienleitung für die Zufuhr von Druckluft zur Reinigungskammer vorgesehen, um zum Beispiel zusätzliche Funktionen ausüben zu können oder aber für Spül-, Wartungs- oder Trocknungszwecke.

In einer Weiterentwicklung der Erfindung ist vorgesehen, dass eine Speichereinheit zur Erfassung und Speicherung der Betriebszeit, der Betriebsdauer, der Medienentnahmezeit, der Medienentnahmemenge, der Medienentnahmedauer, der durch den Sensor erfassten Werte und/oder der Entnahmegeschwindigkeit vorgesehen ist. Durch eine solche optionale, erfindungsgemäße Ausgestaltung wird erreicht, dass die Reinigung der Instrumente nachweisbar ist. Der Benutzer, insbesondere der Zahnarzt, der eine solche erfindungsgemäße Pflegevorrichtung einsetzt, gewinnt damit einen Überblick über die Hygienequalität, aber auch den Wartungszustand der von ihm eingesetzten dentaltechnischen Geräte und Instrumente, was beispielsweise im Zuge einer Zertifizierung erhebliche Vorteile mit sich bringt.

Neben anderen Werten ist auch die Entnahmegeschwindigkeit hierbei von Interesse. Die Entnahmegeschwindigkeit (oder auch Entnahmerate) ist dabei definiert durch den Quotient, des aus dem Vorratsgefäß entnommenen Mediums und der Öffnungszeit des Auslasses, zum Beispiel eines Magnetventils in der Dosiervorrichtung.

Die Erfindung umfasst nicht nur eine Pflegevorrichtung, wie vorstehend beschrieben, sondern die erfindungsgemäße Aufgabe wird auch durch ein Verfahren zur Überwachung des Füllstandes eines insbesondere fluidischen Reinigungs- oder Pflegemediums in einem Vorratsgefäß einer Pflegevorrichtung gelöst, welches folgende Schritte umfasst:

Zunächst wird vor einer Medienentnahme aus einem Vorratsgefäß für das Medium, insbesondere ein Reinigungs-, Desinfektions- und/oder Pflegemedium, sensorgestützt ein in dem Vorratsgefäß vor einer Medienentnahme herrschender statischer Druck erfasst. Es erfolgt dann eine Auswertung der erfassten Werte und danach eine Ableitung des Füllstandes des Mediums im Vorratsgefäß vor der Medienentnahme aus dem Vorratsgefäß. Insbesondere durch die Druckmessung erreicht das erfindungsgemäße Verfahren, dass der von dem Füllstand abhängige Druck als weiterer, auswertbarer Parameter zur Verfügung steht und eben dann eine Vielzahl von weiteren Steuer- und Auswertemöglichkeiten bestehen. Es ist möglich, den ermittelten Füllstand als entsprechende Füllstandsinformation einer Anzeige zuzuleiten und dort zu visualisieren oder eine entsprechende Warnnachricht auszugeben oder als Ausgangsgröße für die Steuerung der Entnahmedauer des Mediums zu verwenden.

Erfindungsgemäß wird dabei weiterhin vorgeschlagen, dass die Auswertung in einer Auswerteinheit erfolgt, wobei anhand der durch den Sensor beziehungsweise die Sensoren erfassten Werte, nämlich anhand des statischen Druckes und/oder einer Druckänderung im Medium eine Berechnung der Medienmenge beziehungsweise des Füllstandes erfolgt.

Nach der Bestimmung des Füllstandes, das heißt dessen Ableitung aus den Sensorwerten beziehungsweise -informationen, wird dann in einem weiteren erfindungsgemäßen Schritt der Füllstand mit einem für die jeweilige Anwendung vorgegebenen Mindestwert verglichen und so sichergestellt, dass ausreichend Medium für die Durchführung des Pflege-/Reinigungsschrittes zur Verfügung steht. Ergibt es sich nämlich, dass vor oder während der Entnahme des Mediums der Füllstand unter dem Mindestwert liegt oder absinken könnte, ist nicht mehr sichergestellt, dass ausreichend Reinigungs-, Desinfektions- und/oder Pflegemittel zu dem dentaltechnischen Gerät geleitet wird, woraus dann eine entsprechend unvollständige Reinigung, Desinfizierung oder auch Pflege beziehungsweise Wartung resultieren könnte. In einer solchen Situation ist erfindungsgemäß vorgesehen, dass ein insbesondere optisches oder akustisches Signal beispielsweise eine akustische Nachfüllaufforderung ausgegeben wird oder eine Sperrung des Vorratsgefäßes oder der Medienleitung erfolgt oder aber die Dosiervorrichtung deaktiviert wird.

Des Weiteren umfasst die Erfindung natürlich auch die Variante, dass mit Hilfe des Sensors die Entnahmemenge beziehungsweise die Entnahmegeschwindigkeit des Reinigungs- oder Pflegemediums bestimmt wird, wobei dies während oder nach der Medienentnahme erfolgt. Durch die Ermittlung der Entnahmegeschwindigkeit beziehungsweise Entnahmerate kann dann auf die Entnahmedauer des Mediums aus dem Vorratsgefäß Einfluss genommen werden. Ergibt es sich nämlich, dass der Füllstand im Vorratsgefäß verhältnismäßig gering ist, dann wirkt ein verhältnismäßig geringer Druck auf die Mediumsäule in der Mediumleitung vom Vorratsgefäß zur Dosiervorrichtung und die Dosiervorrichtung muss eine längere Zeit in der geöffneten Stellung verbleiben, um eine ausreichende Mediummenge aus dem Vorratsgefäß zu entnehmen. Es ist daher günstig, dass in Abhängigkeit des Füllstandes die Dosiervorrichtung gesteuert wird, wobei insbesondere bei geringerem Füllstand die Entnahmedauer des Mediums aus dem Vorratsgefäß erhöht wird. Diese Erhöhung erfolgt dabei unter dem Aspekt, dass eine möglichst gleichbleibende Medienmenge für Reinigungs-, Desinfektions-, Spül-, Wartungs- oder Pflegezwecke zur Verfügung steht.

Die Erfindung betrifft des Weiteren in einem zweiten Aspekt oder Teil eine Pflegevorrichtung für wenigstens ein dentaltechnisches Gerät, die ein Vorratsgefäss für ein Medium, insbesondere ein Reinigungs- oder Pflegemedium, wenigstens eine Reinigungskammer für das dentaltechnische Gerät, wenigstens eine das Vorratsgefäß und die Reinigungskammer verbindende Medienleitung und eine Dosier- und Verteilvorrichtung für das Reinigungs- und Pflegemedium umfasst.

Entsprechende Pflegevorrichtungen sind bekannt. Bei herkömmlichen Pflegevorrichtungen ist jedoch zur Entnahme des Mediums aus dem Vorratsgefäß und auch zur Durchleitung durch eine eventuell vorhandene Dosier- und Verteilvorrichtung die Verwendung von Fördervorrichtungen, beispielsweise Förderpumpen oder Saugvorrichtungen, das heißt, Unterdruckpumpen vorgesehen. In der Regel wird hierbei für jedes Medium eine eigene Fördervorrichtung vorgesehen. Dies führt dazu, dass herkömmliche Pflegevorrichtungen besonders aufwändig gestaltet sind. Aufgrund der Verwendung einer Vielzahl von Fördervorrichtungen sind diese Pflegevorrichtungen zum einen in der Herstellung besonders kostenintensiv, zum anderen besteht die Möglichkeit, dass die einzelnen Fördervorrichtungen ausfallen und ggf. ausgetauscht werden müssen, was wiederum mit einem erhöhten Aufwand für die Wartung bzw. Instandhaltung der Vorrichtungen einhergeht.

Hierzu wird gemäß der Erfindung eine, wie kurz vorher angegebene Pflegevorrichtung wie folgt verbessert. Die Pflegevorrichtung ist dadurch gekennzeichnet, dass die Dosier- und Verteilvorrichtung eine für die an der Dosier- und Verteilvorrichtung angeschlossenen von den Vorratsgefäßen herführenden Medienleitungen gemeinsame Aufnahmekammer aufweist und die Aufnahmekammer als Transportleitung Teil der Medienleitung ist und eine schwerkraftbeaufschlagte Überführung des Mediums aus dem Vorratsgefäß in die Aufnahmekammer der Dosier- und Verteilvorrichtung vorgesehen ist. Durch diese Ausgestaltung der erfindungsgemäßen Pflegevorrichtung wird das Problem umgangen, dass hier zusätzliche Fördervorrichtungen für das Medium vorgesehen werden müssen. Dies bietet den Vorteil, dass die Pflegevorrichtung kostengünstig hergestellt werden kann.

Als weiterer Vorteil der erfindungsgemäßen Pflegevorrichtung ist anzusehen, dass hier, aufgrund des Fehlens von entsprechenden Fördervorrichtungen, kein Wartungsaufwand für diese Fördervorrichtungen anfällt, die Pflegevorrichtung somit insgesamt als besonders nutzerfreundlich und wartungsarm anzusehen ist.

Die erfindungsgemäß vorgeschlagene Aufnahmekammer ist entsprechend groß dimensioniert und bildet eine gemeinsame Aufnahmekammer für alle oder für mehrere Medienleitungen, das heißt, das in den mehreren Medienleitungen herangeführte Fluid oder Flüssigkeit gelangt in eine gemeinsame Aufnahmekammer, die Teil der Transportleitung beziehungsweise der Medienleitung zur Reinigungskammer für das dentaltechnische Gerät ist. Eine separate Anordnung von einzelnen Kammern für jedes Vorratsgefäß mit entsprechend komplexer Ventilsteuerung ist hier nicht notwendig, dies kann alles in der gemeinsamen Dosier- und Verteilvorrichtung erfolgen. Diese hat dabei eine zentrale Aufgabe und eine Vielzahl von Anschlüssen beziehungsweise Auslässen zu den einzelnen Aufnahmestutzen beziehungsweise Bearbeitungspunkte für die dentaltechnischen Geräte in der Reinigungskammer. Der erfindungsgemäße Vorschlag zeichnet sich dabei durch seine geringe Kosten für seine Realisierung aus und erlaubt auch eine große Flexibilität im Einsatz, da an der Dosier- und Verteilvorrichtung zum Beispiel eine Mehrzahl von Blindanschlüssen für noch nicht eingesetzte Vorratsgefäße vorsehbar sind, die dann gegebenenfalls problemlos nachgerüstet und adaptiert werden können.

Nachdem das Medium in die Dosier- und Verteilvorrichtung überführt wurde, sieht eine bevorzugte Weiterbildung der erfindungsgemäßen Pflegevorrichtung vor, dass ein Eintrag des Mediums in die Reinigungskammer mittels Druckbeaufschlagung erfolgt. Diese Druckbeaufschlagung kann beispielsweise durch das Anlegen eines Über- oder Unterdruckes an der Dosier- und Verteilvorrichtung erfolgen. Daneben besteht auch die Möglichkeit, dass die Reinigungskammer selbst unterdruckbeaufschlagt und das Medium somit aus der Dosier- und Verteilvorrichtung in die Reingigungskammer eingesaugt wird. Daneben sieht eine weitere mögliche Ausführungsform der erfindungsgemässen Pflegevorrichtung vor, dass die Dosier- und Verteilvorrichtung druckluftbeaufschlagt wird und hierdurch ein Eintrag des Mediums in die Reinigungskammer durchführbar ist. Die erfindungsgemäße Pflegevorrichtung hat somit den Vorteil, dass lediglich eine Fördervorrichtung, die der Reinigungskammer zugeordnet ist, vorzusehen ist. Bei der Verwendung von Druckluft kann auch diese Fördervorrichtung beiseite gelassen werden. Auch das ist mit Blick zum einen auf eine kostengünstige Herstellung und zum anderen auf einen wartungsarmen Betrieb der Pflegevorrichtung als vorteilhaft anzusehen.

Das Medium, das schwerkraftbeaufschlagt in die Dosier- und Verteilvorrichtung überführt wurde, erfährt bevorzugt in der Dosier- und Verteilvorrichtung eine Druckbeaufschlagung zur Weiterleitung bzw. zum Eintrag des Mediums in die Reinigungskammer. Insbesondere ist hierbei in der Dosier- und Verteilvorrichtung eine Regelungsvorrichtung vorgesehen, über die die Höhe der Druckbeaufschlagung festgelegt werden kann. Bei dieser Regelungsvorrichtung kann es sich beispielsweise um ein elektromagnetisches Regelventil handeln. Eine Steuerung des Ventils kann über eine Auswerte- und Steuervorrichtung, wie beispielsweise nachfolgend beschrieben, erfolgen. Eine besonders einfache Ausführungsform der Pflegevorrichtung sieht vor, dass die Regelungsvorrichtung lediglich als Schieber oder als Klappe mit variabler Öffnungsweite ausgeführt ist und hierüber dann eine Regelung der Höhe der Druckbeaufschlagung erfolgt, die wiederum abhängig ist von den Reinigungs- und Pflegeaufgaben, die in der Reinigungskammer erfüllt werden müssen.

Die erfindungsgemässe Pflegevorrichtung weist eine Dosier- und Verteilvorrichtung auf. Diese Dosier- und Verteilvorrichtung umfasst dabei wenigstens eine Transportleitung für das Medium, wobei hierbei ein und dieselbe Transportleitung für Reinigungs- und Pflegemedien verwendet werden kann. In der Transportleitung angeordnet oder der Transportleitung zugeordnet befindet sich darüber hinaus wenigstens ein Zwischendepot für das Medium, das im Zuge der Einleitung des Mediums aus dem Vorratsgefäß in die Dosier- und Verteilvorrichtung mit dem Medium gefüllt wird. Darüber hinaus ist in der Dosier- und Verteilvorrichtung ein Einlass für die Druckbeaufschlagung vorgesehen. Über diesen Einlass kann beispielsweise Druckluft als Transportmedium in die Transportleitung eingeleitet werden, die dann den Eintrag des Mediums in die Reinigungskammer bewirkt. Darüber hinaus besteht auch die Möglichkeit, dass der Einlass dann geöffnet wird, wenn an der Reinigungskammer ein entsprechender Unterdruck anliegt, so dass hier ein Einsaugen des Mediums in die Reinigungskammer erfolgen kann. Neben dem Einlass für die Druckbeaufschlagung weist die Dosier- und Verteilvorrichtung der Transportleitung zugeordnet wenigstens einen Einlass für das Medium auf, wobei hier auch jeweils gesonderte Einlässe für ein Reinigungs- oder auch Desinfektions- und ein Pflegemedium vorgesehen werden können. Um eine Entleerung der Dosier- und Verteilvorrichtung durchführen zu können, weist die Transportleitung zusätzlich einen Auslass für das Medium auf, wobei auch hier wiederum die Möglichkeit besteht, gesonderte Auslässe für Reinigungs- und Pflegemedien vorzusehen. Die Einlässe bzw. Auslässe münden insbesondere in die Transportleitung ein. Der wenigstens eine Einlass für das Medium ist dabei mit dem Vorratsgefäß verbunden, während der wenigstens eine Auslass mit der Reinigungskammer in Verbindung steht. Die Verbindung wird jeweils über die Medienleitung, die in der Pflegevorrichtung vorgesehen ist realisiert. Diese Medienleitung ist nicht nur als einzelne Leitung, sondern auch als Bündel von Leitungen zu verstehen, die mit jeweiligen Einlässen bzw. Auslässen und/oder dem Vorratsgefäss bzw. der Reinigungskammer verbunden sind. Eine bevorzugte und besonders einfach realisierbare Ausführungsform der Pflegevorrichtung sieht vor, dass als Transportmedium Druckluft vorgesehen ist und der entsprechende Einlass für die Druckbeaufschlagung als Einlass für dieses Transportmedium dient und daher mit einer Druckluftleitung oder einem Kompressor verbunden ist.

Die Pflegevorrichtung umfasst erfindungsgemäß eine Reinigungskammer für die Aufnahme oder Anordnung des dentaltechnischen Gerätes. Hierunter sind alle in der zahnärztlichen Praxis verwendeten Geräte oder Instrumente, das heißt deren Handstücke beziehungsweise Anwendungsteile zu verstehen. Die Pflegevorrichtung bleibt jedoch nicht auf die Ausgestaltung mit einer allseitig geschlossenen Kammer beschränkt. Vielmehr kann auch vorgesehen werden, dass es sich bei der Reinigungskammer um eine Aufnahmeschale oder dergleichen handelt, die nur eine umlaufende Seitenwandung aufweist, im übrigen jedoch,beispielsweise nach oben und/oder unten hin offen ist. Daneben besteht auch die Möglichkeit, dass die Reinigungskammer lediglich aus einer Zusammensetzung von Positionierungspunkten für dentaltechnische Geräte besteht, und diese Aufnahmevorrichtung, die somit als Reinigungskammer zu verstehen ist, dann in eine Behältnis, beispielsweise ein Waschbecken oder dergleichen eingesetzt oder eingehängt werden kann.

In einer vorteilhaften Weiterbildung der Erfindung ist vorgesehen, dass in der Reinigungskammer ein Positionierungspunkt für das dentaltechnische Gerät vorgesehen ist. Dieser Positionierungspunkt kann insbesondere in Form eines Anschlussstutzens ausgebildet sein, mit dem das dentaltechnische Gerät verbunden, beispielsweise auf diesem aufgeschraubt, aufgesetzt oder mit diesem verrastet wird. Desweiteren ist vorgesehen, dass wenigstens ein Auslass für das Medium mit dem Positionierungspunkt verbunden ist und über den Positionierungspunkt eine Anbindung des Auslasses an das dentaltechnische Gerät bzw. ein im dentaltechnischen Gerät vorgesehenes Leitungssystem beziehungsweise eine Öffnung des Auslasses erfolgt. Bei dem Leitungssystem kann es sich um das ohnehin im dentaltechnischen Gerät vorgesehene Leitungssystem für zahnärztliche Behandlungsmedien, wie beispielsweise Wasser, Desinfektionslösung für den Mundraum oder Druckluft handeln, das dann dazu benutzt wird, das Reinigungs- oder Pflegemedium für das Gerät durch das Gerät zu leiten, um hierbei Reinigungs- oder Pflegeaufgabe zu erfüllen.

Eine bevorzugte Ausführungsform der Pflegevorrichtung sieht in diesem Zusammenhang vor, dass eine Anbindung beziehungsweise Öffnung des entsprechenden Auslasses in der Reinigungskammer nur bei der Positionierung eines dentaltechnischen Gerätes erfolgt. Um diese Ausführungsform zu realisieren, besteht die Möglichkeit, dass in dem Positionierungspunkt beziehungsweise im Anschlussstutzen ein mechanisches Ventil integriert wird, das nur bei Aufsetzen oder Ansetzen beziehungsweise Verrasten eines dentaltechnischen Gerätes mit dem Anschlussstutzen geöffnet wird. In einer aufwändigeren Ausgestaltungsform ist vorgesehen, dass im Positionierungspunkt ein Erkennungssensor vorgesehen ist, der dann wiederum rückkoppelnd über eine Steuerungsvorrichtung, eine Sperrvorrichtung, beispielsweise ein Magnetventil oder dergleichen, in der Dosier- und Verteilvorrichtung ansteuert und hierüber dann der Auslass freigegeben beziehungsweise eine entsprechende Sperrvorrichtung geöffnet wird, sodass das Medium in das dentaltechnische Gerät einströmen kann.

In der Dosier- und Verteilvorrichtung beziehungsweise dieser zugeordnet, ist vorteilhafterweise wenigstens eine erste Sperrvorrichtung vorgesehen, die zur Unterteilung der Transportleitung dient. Diese Sperrvorrichtung kann dabei insbesondere in Form eines elektronischen Magnetventils ausgebildet sein und über die Steuerungsvorrichtung oder aber programmgesteuert geöffnet oder geschlossen werden. In einer einfacheren Ausführungsform der erfindungsgemäßen Pflegevorrichtung ist die Sperrvorrichtung als einfache Klappe oder Schieber ausgebildet, die in die Transportleitung eingeschoben oder in dieser verschwenkt wird, um die geforderte Unterteilung durchzuführen. Ziel der Unterteilung der Transportleitung ist es, eine Zuleitung unterschiedlicher Medien zu verschiedenen Auslässen durchführen zu können. So kann beispielsweise, wie in einer weiteren bevorzugten Ausführungsform der erfindungsgemäßen Pflegevorrichtung vorgesehen, in der Dosier- und Verteilvorrichtung ein erstes Zwischendepot für ein erstes Medium, beispielsweise ein Reinigungsmedium, dem Einlass für die Druckbeaufschlagung beziehungsweise das Transportmedium unmittelbar nachgeordnet angeordnet sein. Diesem Zwischendepot nachgeordnet befinden sich dann eine Vielzahl von Auslässen, die mit der Reinigungskammer verbunden sind. Wird nun das erste Zwischendepot mit dem Reinigungsmedium befüllt und die Dosier- und Verteilvorrichtung anschließend druckbeaufschlagt, so erfolgt der Austritt des Reinigungsmediums aus allen Auslässen. Es kann somit erreicht werden, dass alle dentaltechnischen Geräte mit Reinigungsmedium beaufschlagt und dadurch gereinigt oder desinfiziert werden. Ein weiteres Zwischendepot kann beabstandet zum Einlass für die Druckbeaufschlagung beziehungsweise das Transportmedium in der Dosier- und Verteilvorrichtung angeordnet sein. Diesem zweiten Zwischendepot zugeordnet kann dann die erste Sperrvorrichtung für die Transportleitung in der Dosier- und Verteilvorrichtung vorgesehen werden. Dem Zwischendepot ist wiederum ein Einlass für ein weiteres Medium, beispielsweise ein Pflegemedium zugeordnet. Wird nun die Sperrvorrichtung geschlossen und erfolgt danach eine Befüllung des zweiten Zwischendepots mit dem Pflegemedium, so kann nach erneutem Öffnen der Sperrvorrichtung und Druckbeaufschlagung der Dosier- und Verteilvorrichtung ein Eintrag des Pflegemedium nur in die dem zweiten Zwischendepot nachgeordneten Auslässe erfolgen. Durch diese Ausführungsform der Dosier- und Verteilvorrichtung können unterschiedliche Reinigungs- beziehungsweise Pflegeaufgaben in ein und demselben Gerät realisiert werden, da eine zeitweise oder bedarfsweise Absperrung von Bereichen der Dosier- und Verteilvorrichtung durchgeführt werden kann. Neben dieser Ausgestaltung besteht selbstverständlich auch die Möglichkeit, dass ein weiterer Einlass für eine Druckbeaufschlagung in der Dosier- und Verteilvorrichtung vorgesehen ist, die dann dem zweiten Zwischendepot beziehungsweise dem zweiten Einlass für das weitere Medium, beispielsweise das Pflegemedium, zugeordnet ist und hierbei eine Druckbeaufschlagung nur des durch die Sperrvorrichtung abgetrennten Bereichs der Dosier- und Verteilvorrichtung durchgeführt wird.

Um flexibel auf die Auslastung der Reinigungskammer reagieren zu können und/oder um verschiedene Reinigungsprogramme beziehungsweise den Eintrag unterschiedlicher Medien in verschiedene dentaltechnische Geräte steuern zu können, sieht eine als günstig erachtete Weiterbildung der Erfindung vor, dass jeweils eine dem Einlass für das Transportmedium und/oder dem Einlass oder dem Auslass für das Medium zugeordnete weitere Sperrvorrichtung vorgesehen ist. Über diese Sperrvorrichtung(en) kann dann eine gesteuerte Druckbeaufschlagung einerseits und der Austritt von Medium aus der Dosier- und Verteilvorrichtung andererseits geregelt werden. Auch die weitere(n) Sperrvorrichtung(en) kann/können als elektronische(s) Magnetventil(e) beziehungsweise als Klappe(n) oder als Schieber ausgebildet sein. Die Ausgestaltung als elektronisches Magnetventil erlaubt die Anbindung der Sperrvorrichtung(en) an eine Steuerungsvorrichtung, sodass hier insbesondere programmgesteuert ein Öffnen und Schließen des Auslasses beziehungsweise der Auslässe und der entsprechenden Einlässe durchgeführt werden kann. Werden lediglich Klappen oder Schieber als Sperrvorrichtung verwendet, so kann auch hier eine manuelle Öffnung beziehungsweise Schließung von Ein- und Auslässen erfolgen.

Die Pflegevorrichtung umfasst vorteilhafterweise auch eine Steuervorrichtung, die zur Steuerung der einzelnen Sperrvorrichtungen verwendet werden kann. Die Steuervorrichtung weist insbesondere wenigstens ein hinterlegtes Steuerungsprogramm auf, wodurch insbesondere eine Steuerung der Öffnungs- und Verschlusszeitpunkte und/oder der Öffnungs- und Verschlussabfolge sowie der Öffnungs- und Verschlussdauer der Sperrvorrichtungen beeinflusst wird. Die Steuervorrichtung wiederum kann mit einem beispielsweise in der Reinigungskammer vorgesehenen Erkennungssensor verbunden werden und nur bei der Verwendung eines entsprechenden Positionierungspunktes die zugehörige Sperrvorrichtung am Auslass für das Medium aktivieren, das heißt öffnen und schließen. Aufgrund der Möglichkeit über das Steuerungsprogramm die Öffnungs- und Verschlussdauer der Sperrvorrichtungen an den Einlässen für das Medium zu regulieren, kann eine vom Füllstand der Vorratsgefäße abhängige Befüllung des Zwischendepots in der Dosier- und Verteilvorrichtung durchgeführt werden. Da dieses Einleiten des Mediums aus den Vorratsgefäßen in die Verteil- und Dosiervorrichtung ausschließlich schwerkraftbeaufschlagt erfolgen soll, unterliegt die Befüllung Schwankungen, die abhängig sind von dem Füllstand im Vorratsgefäß. Ist im Vorratsgefäß viel Medium vorhanden, so wird dieses aufgrund des höheren anliegenden Druckes schneller aus dem Vorratsgefäß austreten als beispielsweise bei einem niedrigeren Füllstand, der mit einem langsameren Ausfließen des Mediums einhergeht. Um sicherzustellen, dass stets die richtige und ausreichende Menge an Medium aus dem Vorratsgefäß entnommen wird, erfolgt eine Rückkopplung zwischen der Steuervorrichtung und einer Füllstandserfassung am Vorratsgefäß. Ausgehend von dem ermittelten Füllstand wird dann die Öffnungsdauer der Sperrvorrichtung am Einlass für das Medium verändert und damit eine stets gleichmäßige Befüllung des Zwischendepots sichergestellt.

Eine Weiterbildung der erfindungsgemäßen Pflegevorrichtung sieht vor, dass, wie bereits ausgeführt, eine Betätigung der dem Einlass für das Medium zugeordneten Sperrvorrichtung anhand von für den Füllstand des Mediums im Vorratsgefäß ermittelten Werten erfolgt. Insbesondere ist hierbei vorgesehen, über entsprechende Sensoren eine Erfassung von Änderungen der elektrischen Kapazität und/oder einer Schwimmerposition im Medium oder aber eine Ermittlung von Druckwerten durchzuführen, wozu bevorzugt ein dem Vorratsgefäß zugeordneter elektronischer Drucksensor verwendet wird. Daneben ist eine Vielzahl von sonstigen Verfahren zur Füllstandsbestimmung bekannt. So besteht beispielsweise die Möglichkeit, die Füllstandskontrolle über eine Leitfähigkeitsmessung, eine optische Messung oder eine Ultraschall- oder Mikrowellenmessung durchzuführen. Die Füllstandsermittlung bleibt jedoch nicht auf die genannten Messverfahren beziehungsweise Vorrichtung zur Ausführung der entsprechenden Messverfahren beschränkt, sondern umfasst alle entsprechend verwendbaren oder einsetzbaren Vorrichtungen und Verfahren zur genauen Bestimmung des Füllstandes.

In der Auswert- und Steuervorrichtung kann auch eine Ermittlung des Medienbedarfs für die Durchführung eines Pflege- oder Reinigungsverfahrens ermittelt werden. Dies erfolgt auch beispielsweise über die der Auswert- und Steuervorrichtung mitgeteilte Anzahl an belegten Positionierungspunkten beziehungsweise über das gewählte Reinigungsprogramm, sofern vorgesehen. Aus den so ermittelten beziehungsweise aufgenommenen Parametern kann die Auswert- und Steuervorrichtung beziehungsweise das Steuerungsprogramm den Bedarf an Medium ermitteln und diesen mit dem in den Vorratsgefäßen vorhandenen Füllständen vergleichen. Wird nun festgestellt, dass ein kritischer Füllstand beziehungsweise ein Mindestfüllstand für das Medium in einem der Vorratsgefäße unterschritten wird, so kann durch die Auswert- und Steuervorrichtung beziehungsweise durch das Steuerungsprogramm ein entsprechendes Signal (optisch oder akustisch) ausgegeben werden, und die Pflegevorrichtung ansonsten bis zur Behebung des Problems, das heißt bis zur Nachfüllung von Medium gesperrt werden.

Als vorteilhaft wird angesehen, wenn das in der Dosier- und Verteilvorrichtung angeordnete Zwischendepot im Bereich des Einlasses für das Medium angeordnet ist. Hierdurch wird vermieden, dass das Medium durch die gesamte Dosier- und Verteilvorrichtung beziehungsweise durch Bereiche der Vorrichtung transportiert werden muss, bevor diese über die entsprechenden Auslässe in die Reinigungskammer eingetragen wird.

Das Zwischendepot kann vorteilhafterweise als Becken oder Rinne in der Transportleitung ausgebildet sein. Darüberhinaus besteht die Möglichkeit, dass an einem Ende der Transportleitung eine Aufweitung angeordnet ist, die als Zwischendepot dient. Als weitere, besonders einfache Ausführung beziehungsweise Möglichkeit zur Bildung eines Zwischendepots wird angesehen, wenn die Transportleitung in der Dosier- und Verteilvorrichtung bezüglich der Gebrauchsstellung der Pflegevorrichtung, das heißt beispielsweise gegebenüber der Horizontalen geneigt eingebracht ist und ein insbesondere verschlossenes Ende der Transportleitung als Zwischendepot dient. Das Medium wird dabei über den Einlass in die Dosier- und Verteilvorrichtung eingebracht und fließt dann zum niedrigsten Punkt in der Transportleitung, wo es sich sammelt, um von dort aus dann über die Auslässe in die Reinigungskammer beziehungsweise das dort angeordnete dentaltechnische Gerät eingetragen zu werden.

Im Interesse einer späteren Aufrüstbarkeit beziehungsweise Erweiterbarkeit der Pflegevorrichtung erweist es sich als günstig, wenn die Dosier- und Verteilvorrichtung als Modul ausgebildet beziehungsweise aus wenigstens einem derartigen Modul aufgebaut ist. Das Modul umfasst dabei wenigstens einen Abschnitt der Transportleitung, wenigstens ein Zwischendepot, wenigstens einen Einlass für die Druckbeaufschlagung, das heißt für die Zuleitung eines Transportmediums oder aber die Unterdruckbeaufschlagung des im Zwischendepot gesammelten Mediums, wenigstens einen Einlass und wenigstens einen Auslass für Medium. Es kann somit beispielsweise in einer Grundkonfiguration eine Pflegevorrichtung für ein einzelnes dentaltechnisches Gerät angeboten werden. Steigt nun der Reinigungsbedarf, beispielsweise durch die Anschaffung weiterer Geräte oder durch die Erweiterung der zahnärztlichen Praxis, so kann die Anschaffung weiterer Pflegevorrichtungen dadurch umgangen werden, dass ein zusätzliches Modul an das bereits vorhandene Modul mit einer ersten Dosier- und Verteilvorrichtung angesetzt wird und die Dosier- und Verteilvorrichtung dadurch erweitert wird. Diese Erweiterung kann somit auf kostengünstige, einfache und schnelle Weise realisiert werden.

Als günstig wird angesehen, wenn das entsprechende Modul dadurch weitergebildet wird, dass hier ebenfalls eine Sperrvorrichtung für die Transportleitung und/oder weitere Sperrvorrichtungen für den Einlass für das Transportmedium beziehungsweise die Druckbeaufschlagung und/oder den Einlass oder den Auslass für das Medium vorgesehen sind. Hierüber kann dann eine direkte Anbindung des jeweils hinzugekommenen Moduls an die Steuervorrichtung beziehungsweise eine Integration in das Steuerprogramm erfolgen und die Pflegevorrichtung somit auch steuerungstechnisch erweitert werden.

Eine als günstig angesehene Ausführungsform der erfindungsgemäßen Pflegevorrichtung sieht vor, dass die Dosier- und Verteilvorrichtung als austauschbarer Block ausgeführt ist. Dies bietet Vorteile insbesondere bei der Wartung, Instandhaltung oder Reinigung der Pflegevorrichtung, da dadurch die Dosier- und Verteilvorrichtung als einzelner Systembaustein aus der Gesamtvorrichtung entnommen, gereinigt, inspiziert oder ausgetauscht werden kann, ohne hierbei einen besonders hohen Wartungs- oder Reparaturaufwand zu erzeugen.

Die Erfindung umfasst neben der bereits beschriebenen Vorrichtung auch ein Verfahren zum Reinigen und/oder Pflegen wenigstens eines dentaltechnischen Gerätes. Das Verfahren verwendet dabei insbesondere eine, wie oben bereits beschrieben, Pflegevorrichtung. Das Reinigungs- und/oder Pflegeverfahren umfasst die folgenden Schritte. Zunächst erfolgt die ausschließlich schwerkraftbeaufschlagte Einleitung eines Mediums, insbesondere eines Reinigungs- oder Pflegemediums aus einem Vorratsgefäß in eine Dosier- und Verteilvorrichtung. Das in die Dosier- und Verteilvorrichtung eingeleitete Medium wird in der Dosier- und Verteilvorrichtung zwischenbevorratet. Diese Zwischenbevorratung ist notwendig aufgrund der ausschließlich schwerkraftbeaufschlagten Einleitens des Mediums in die Dosier- und Verteilvorrichtung, da aufgrund unterschiedlicher Druckverhältnisse, die im Vorratsgefäß herrschen, ein unterschiedlich schnelles Ausfließen des Mediums aus dem Vorratsgefäß stattfindet. Um nun eine definierte Menge an Medium aus dem Vorratsgefäß zu entnehmen, sind unterschiedliche Einleitungszeiten notwendig. Somit empfiehlt sich die Zwischenbevorratung des Mediums in entsprechenden Zwischendepots der Dosier- und Verteilvorrichtung, um hier stets eine gleichbleibende Medienmenge für die Reinigung beziehungsweise Pflege des dentaltechnischen Gerätes, das heißt beispielsweise der Handstücke und Anwendungsteile zur Verfügung stellen zu können. Als weiterer Schritt des Verfahrens ist vorgesehen, die Dosier- und Verteilvorrichtung mit Druck zu beaufschlagen, so dass ein Eintrag des dort zwischenbevorrateten Mediums in die Reinigungskammer beziehungsweise in das dort angeordnete dentaltechnische Gerät durchgeführt werden kann.

Eine Weiterbildung des erfindungsgemäßen Verfahrens sieht vor, dass ein gesteuertes Einleiten des Mediums in die Dosier- und Verteilvorrichtung erfolgt. Hierzu wird die Einleitungsdauer und/oder die Einleitungsmenge anhand des Füllstandes des Mediums im Vorratsgefäß ermittelt. Eine derartige Füllstandsermittlung kann insbesondere anhand von Druckwerten, die im Vorratsgefäß herrschen, ermittelt werden. Darüber hinaus stehen jedoch alle vorstehend bereits beschriebenen Möglichkeiten zur Ermittlung des Füllstandes zur Verfügung, anhand derer dann festgelegt wird, wie viel beziehungsweise wie lange eine Einleitung des Mediums in die Dosier- und Verteilvorrichtung erfolgt. Die Einleitungsdauer und/oder Einleitungsmenge kann jedoch auch in Anpassung an das zu reinigende oder zu pflegende dentaltechnische Gerät festgelegt werden. Ebenfalls denkbar ist eine Festlegung anhand der Anzahl von zu reinigenden oder zu pflegenden dentaltechnischen Geräten.

Günstigerweise sind in der Dosier- und Verteilvorrichtung Sperrvorrichtungen für Einlässe und/oder Auslässe und eine Transportleitung für das Medium vorgesehen. Das erfindungsgemäße Verfahren sieht im Zusammenhang mit diesen Sperrvorrichtungen vor, dass deren programmgesteuerte Betätigung durchgeführt wird, wobei insbesondere die Öffnungs- und Verschlusszeitpunkte, die Öffnungs- und Verschlussdauer und die Öffnungs- und Verschlussabfolge der Sperrvorrichtungen programmgesteuert durchgeführt wird. Durch diese Konfiguration des Verfahrens besteht die Möglichkeit zu einer zielgenauen Einleitung beziehungsweise Zuleitung von Medium zu den jeweiligen Auslässen beziehungsweise andererseits auch die gesteuerte Einleitung von Medium in die Dosier- und Verteilvorrichtung, so dass hier eine Vielzahl von unterschiedlichen Pflege- und Wartungs- beziehungsweise Reinigungsaufgaben mit ein und derselben Dosier- und Verteilvorrichtung programmgesteuert durchgeführt werden kann.

Die Durchführung verschiedener Reinigungs- und Pflegeprogramme wird dadurch vereinfacht, dass vorgesehen ist, eine Unterteilung der Transportleitung durchzuführen, so dass nur die einer entsprechenden Sperrvorrichtung in der Transportleitung vorbeziehungsweise nachgeordneten Auslässe mit dem entsprechenden Medium in Kontakt treten können. Eine derartige Unterteilung der Transportleitung wird ebenfalls programmgesteuert durch die Betätigung einer Sperrvorrichtungen in der Transportleitung durchgeführt. Wird eine derartige Abtrennung oder Unterteilung der Transportleitung durchgeführt bevor das entsprechende Medium in die Transportleitung eingeleitet wird, so kann hierüber erreicht werden, dass das Medium nur in den abgetrennten Bereich beziehungsweise in einen abgetrennten Bereich der Transportleitung eingeleitet und aus diesem wiederum nur auf bestimmte Auslässe verteilt und in die Reinigungskammer eingeleitet wird. Dieses Verfahren bietet sich beispielsweise dann an, wenn in der Reinigungskammer sowohl Handgeräte, die gegebenenfalls lediglich einer Pflege bedürfen, neben zahntechnischen Instrumenten oder Anwendungsteilen angeordnet sind, die zugleich eine Reinigung oder Desinfektion benötigen. Hier kann durch die entsprechend geschickte Unterteilung der Transportleitung zunächst nur ein Reinigungsmedium zu den entsprechend zu reinigenden Instrumenten zugeleitet werden, während nach Aufhebung der Unterteilung ein Pflegemedium zu allen Geräten geführt werden kann, um diese entsprechend zu warten oder zu pflegen. Daneben besteht auch die Möglichkeit, einen Teil der Dosier- und Verteilvorrichtung zu sperren, wenn nur ein Teil der Positionen in der Reinigungskammer mit zu reinigenden und/oder zu pflegenden Instrumenten oder Geräten belegt ist. Hierdurch kann eine Einsparung von Reinigungs- und Pflegemedium erreicht werden.

Eine vorteilhafte Weiterbildung des erfindungsgemäßen Verfahrens sieht vor, dass die Menge an eingeleitetem beziehungsweise zwischenbevorratetem Medium ebenso wie die Höhe der Druckbeaufschlagung zum Eintrag des Mediums anhand der zu reinigenden oder zu pflegenden dentaltechnischen Geräte eingestellt wird. Dies verlangt wiederum Informationen über die dentaltechnischen Geräte'zur Weitergabe an das Steuerprogramm beziehungsweise die Steuervorrichtung. Dies kann dadurch erfolgen, dass die Informationen in die Steuervorrichtung manuell eingegeben wird, um hier zielgerichtet und klar nachvollziehbar eine angepasste Reinigung oder Pflege der dentaltechnischen Geräte durchzuführen.

In diesem Zusammenhang wird insbesondere darauf hingewiesen, dass alle im Bezug auf die Pflegevorrichtung beschriebenen Merkmale und Eigenschaften aber auch Verfahrensweisen sinngemäß auch bezüglich der Formulierung des erfindungsgemäßen Verfahrens übertragbar und im Sinne der Erfindung einsetzbar und als mitoffenbart gelten. Gleiches gilt auch in umgekehrter Richtung, das bedeutet, nur im Bezug auf das Verfahren genannte, bauliche also vorrichtungsgemäße Merkmale können auch im Rahmen der Vorrichtungsansprüche berücksichtigt und beansprucht werden und zählen ebenfalls zur Erfindung und zur Offenbarung.

In der Zeichnung ist die Erfindung insbesondere jeweils in einem Ausführungsbeispiel schematisch dargestellt. Es zeigen:
- Fig. 1, Fig. 2: Eine schematische Übersicht über den Aufbau je einer erfindungsgemäßen Pflegevorrichtung,
- Fig. 3 bis 5: bevorzugte Ausführungsformen einer in der erfindungsgemäßen Pflegevorrichtung nach Fig. 2 verwendbaren Dosier- und Verteilvorrichtung jeweils in seitlicher Schnittdarstellung.

In den Figuren sind gleiche oder einander entsprechende Elemente jeweils mit den gleichen Bezugszeichen bezeichnet und werden daher, sofern nicht zweckmäßig, nicht erneut beschrieben.

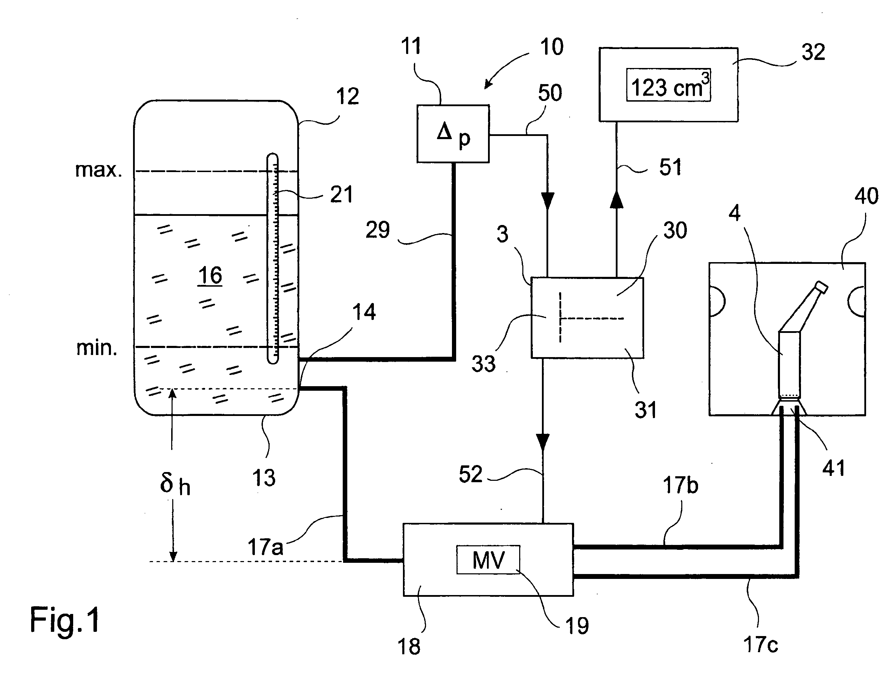

Die erfindungsgemäße Pflegevorrichtung nach Fig. 1 besteht zum einen aus einer Reinigungskammer 40, in welche das zu reinigende beziehungsweise das zu pflegende dentaltechnische Gerät oder Instrument 4 auf einem Sockel 41 aufgesteckt ist. In dem dentaltechnischen Gerät 4 sind Medienleitungen vorgesehen, die die für den Betrieb des dentaltechnischen Gerätes 4 notwendigen Medien (zum Beispiel Druckluft oder Spülwasser und so weiter) in den Bearbeitungskopf leiten. Eine Aufgabe der gattungsgemäßen Pflegevorrichtungen ist es, über diese in dem dentaltechnischen Gerät 4 vorgesehenen Medienleitungen eine Reinigung des Gerätes 4 zu erreichen. Dabei wird mit der erfindungsgemäßen Pflegevorrichtung nicht nur eine innerliche Reinigung des dentaltechnischen Gerätes 4 durchgeführt, optional wird das dentaltechnische Gerät oder Instrument 4 auch äußerlich gereinigt beziehungsweise desinfiziert. Gegebenenfalls sind dann in der Reinigungskammer 40 entsprechende Sprühköpfe und so weiter vorgesehen.

Um die Reinigungskammer 40 mit entsprechenden Reinigungs- und Pflegemedien beziehungsweise -mitteln zu versorgen, werden diese in einem oder mehreren, gegebenenfalls voneinander getrennten Vorratsgefäßen 12 vorgehalten. So ist in den verschiedenen Vorratsgefäßen 12 dann jeweils ein unterschiedliches Reinigungs- beziehungsweise Pflegemittel bzw. Medium 16 vorgehalten, die alle in analoger Weise über eine Medienleitung 17a mit einer (zentralen) Dosiervorrichtung 18 verbunden sind.

Die Ausgestaltung der Dosiervorrichtung 18 ist dabei sehr variabel, sie umfasst zum Beispiel ein elektromagnetisch betätigbares Magnetventil 19 für die jeweiligen Medienzuleitungen 17a.

Gemäß der Erfindung wird bei der hier vorgestellten Pflegevorrichtung der Einsatz eines Drucksensors 11 in der Messeinrichtung 10 vorgesehen. Über den Drucksensor 11 erfolgt die Erfassung von Werten, aus denen dann ein im Vorratsgefäß 12 vorhandener Füllstand eines Mediums 16 abgeleitet wird. Exemplarisch wird hier das Vorgehen beziehungsweise die Ausstattung der erfindungsgemäßen Pflegevorrichtung mit einem Drucksensor 11 beschrieben. Anstelle eines Drucksensors 11 besteht jedoch auch die Möglichkeit, in der Pflegevorrichtung einen kapazitiven Sensor oder einen Schwimmsensor anzuordnen, wobei aus den durch diese Sensoren erfassten Werte ebenfalls die Ableitung eines Füllstandes und die nachfolgende Steuerung der Abgabe von Medium in die Dosiervorrichtung erfolgt. Es wird nochmals darauf hingewiesen, dass die Erfindung nicht auf die Verwendung eines Drucksensors 11 beschränkt bleibt, sondern zur Füllstandsermittlung Werte herangezogen werden, die durch alle geeignet erscheinenden Sensoren, die gleichermaßen in der erfindungsgemäßen Pflegevorrichtung zum Einsatz kommen können, erfasst werden. Denkbar ist hier beispielsweise die Verwendung von weiteren Füllstandskontrollvorrichtungen. Hier kann beispielsweise eine Füllstandskontrolle über eine Leitfähigkeitsmessung, eine optische Messung oder eine Ultraschall- oder Mikrowellenmessung durchgeführt werden. Entsprechende Sensoren oder Erfassungsmittel können dabei in oder an dem Vorratsgefäß 12 oder im Medium 16 angebracht werden und ebenfalls rückkoppelnd mit der Auswerte- und Steuereinheit 30 der erfindungsgemäßen Pflegevorrichtung verbunden sein. Um die Füllstandsmessung weiter zu verbessern, kann auch eine Kombination mehrerer Messverfahren erfolgen, da diese Messverfahren und Vorrichtungen jeweils unterschiedliche Messoptima aufweisen und so durch die Kombination beziehungsweise die Ergänzung der einzelnen Messverfahren und Vorrichtungen eine wesentlich verbesserte und genauere Bestimmung des Füllstandes und damit eine noch besserere Medienentnahme beziehungsweise Füllstandsbestimmung möglich wird. Die Füllstandsermittlung mit sonstigen Verfahren und Vorrichtungen bleibt nicht auf die vorgenannten beschränkt, sondern umfasst alle entsprechend verwendbaren oder einsetzbaren Vorrichtungen und Verfahren, die hier als geeignet erscheinen.

Der Drucksensor 11 ist über die Druckleitung 29 mit dem unteren Bereich des Vorratsgefäßes 12 verbunden. Die Druckleitung 29 kann aber auch am Boden 13 des Gefäßes 12 enden. An dem Vorratsgefäß 12 selbst ist gegebenenfalls eine Füllstandsanzeige 21, hier zum Beispiel als Skala realisiert, vorgesehen. Diese dient zum Beispiel für eine einfache Bestimmung des nachzufüllenden, fehlenden Mediums. Eine solche Information kann aber gegebenenfalls auch über die erfindungsgemäß vorgesehene Auswerteinheit 30 beziehungsweise Steuereinheit 31 ausgegeben werden.

Der mit dem Drucksensor 11 gemessene Druck wird über die Datenleitung 50 zur Auswerteinheit 30 geleitet. Die Auswerteinheit 30 ist dabei gegebenenfalls Teil einer, mehrere Funktionen umfassenden Gerätesteuerung 3. Die Funktion der Auswerteinheit 30 kann aber auch von dem Drucksensor 11 direkt erbracht werden, hierauf ist die Erfindung nicht beschränkt. Die Auswerteinheit 30 ermittelt aufgrund des Drucksignales gegebenenfalls den herrschenden statischen Druck im Vorratsgefäß 12 und davon abgeleitet das noch zur Verfügung stehende Medienvolumen, das gegebenenfalls dann auch auf einer Anzeige 32 angezeigt wird. Hierzu ist die Anzeige 32 mit der Datenleitung 51 mit der Gerätesteuerung 3, hier insbesondere der Auswerteinheit 30 verbunden.

Bei der Gerätesteuerung 3 ist gestrichelt eine interne Unterteilung angedeutet. Dies soll zum Ausdruck bringen, dass die Gerätesteuerung 3 zum Beispiel die Auswerteinheit 30, die Steuereinheit 31 und die Speichereinheit 33 optional beinhaltet. Tatsächlich gelöst ist dies in einer entsprechend speicherprogrammierbaren Steuerung oder einer microcontrolerbasierten Steuerung, die einzelnen Einheiten sind durch entsprechende Programmteile abgedeckt.

Ein wesentlicher Vorzug der Erfindung liegt darin, dass der von der Auswerteinheit 30 ermittelte Wert des Füllstandes mit einem Mindestfüllstand verglichen werden kann und, bei einem Unterschreiten dieses Mindestfüllstandes, zum Beispiel über die Datenleitung 52, die Steuereinheit 31 der Abgabe von Reinigungs- beziehungsweise Pflegemittel/medium in der Dosiervorrichtung 18 unterbindet. Zum Beispiel wird das Magnetventil 19 geschlossen gehalten oder ein zentraler Auslass 14 nicht geöffnet. Gleichzeitig wird ein entsprechendes Warnsignal über die Anzeige 32 (akustisch oder optisch) ausgegeben und natürlich die weitere Bearbeitung des Pflege- beziehungsweise Reinigungs- oder Wartungsprozesses für das dentaltechnische Gerät 4 unterbrochen.

Mit Hilfe der Speichereinheit 33 kann der Nachweis geführt werden über das kontinuierliche Pflegen der dentaltechnischen Geräte 4. Hierzu werden in der Speichereinheit 33 zum Beispiel der Füllstand im Vorratsgefäß 12, insbesondere vor und nach der Medienentnahme, die Medienentnahmemenge, aber auch der Zeitpunkt der Unterschreitung eines Mindestwertes für den Füllstand, der Zeitpunkt der Sperrung des Auslasses 14 oder der Dosiervorrichtung, sowie die Betriebszeit selber, die Betriebsdauer, die Medienentnahmezeit, die Medienentnahmemenge, die Medienentnahmedauer, die erfassten Druckwerte und gegebenenfalls die Entnahmegeschwindigkeit insbesondere laufend und kontinuierlich mitprotokolliert und mitdokumentiert.

Hierin erschöpft sich aber der Effekt der Erfindung nicht. Mit δH ist ein Höhenunterschied zwischen dem Auslass 14 des Gefäßes 12, der in die Medienleitung 17a mündet und der Lage der Dosiervorrichtung 18 gezeigt. Diese Höhendifferenz bewirkt aufgrund der herrschenden Schwerkraft ein Ausfließen des flüssigen Reinigungs- beziehungsweise Pflegemittels von dem Vorratsgefäß 12 über die Medienleitung 17a in Richtung der Dosiervorrichtung 18. Die hierbei resultierende Fließgeschwindigkeit ist allerdings abhängig von dem Druck im Medium und somit in letzter Konsequenz von dem Füllstand im Vorratsgefäß 12. Nun ist die Anordnung so getroffen, dass bei verhältnismäßig geringem Füllstand im Vorratsgefäß 12 und daraus resultierendem geringem Druck die Entnahmedauer der Dosiervorrichtung 18 derart erhöht wird, damit eine ausreichende Entnahmemenge für die Reinigungskammer 40 zur Verfügung steht. Dies wird ebenfalls durch die Steuereinheit 31 gesteuert, die bei entsprechend geringerem Druck, welcher ihr von der Auswerteinheit 30 zur Verfügung gestellt wird, die Entnahmedauer entsprechend erhöht.

Die erfindungsgemäße Variante erlaubt es, eine separate Pumpe für das Entnehmen des Mediums 16 aus dem Vorratsgefäß 12 einzusparen.

Von der Dosiervorrichtung 18 zur Reinigungskammer 40 sind zwei Medienleitungen 17b und 17c gezeigt. Mit Hilfe der Medienleitung 17c wird die Reinigungskammer beziehungsweise der Sockel 41 und insbesondere das dentaltechnische Gerät 4 mit Druckluft beaufschlagt. Dies erfolgt gegebenenfalls getrennt von der Zuleitung des Mediums 16 mit der Medienleitung 17b. Die Förderung des Mediums in der Medienleitung 17b erfolgt dabei zum Beispiel durch eine Pumpe unterstützt oder mit Hilfe der zur Verfügung stehenden Druckluft.

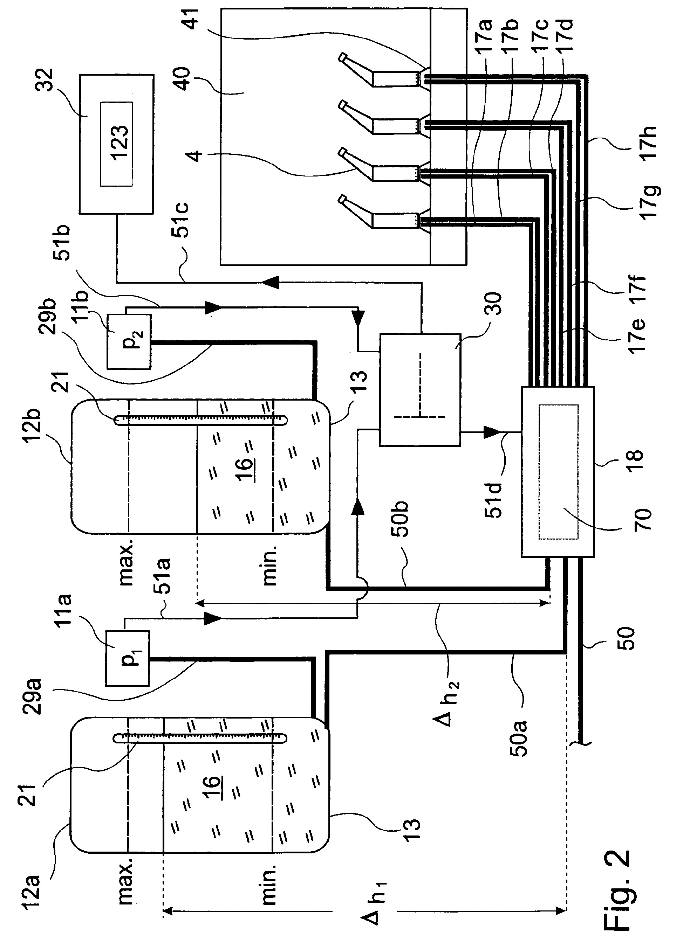

Die erfindungsgemäße Pflegevorrichtung nach Fig. 2 besteht zum Einen aus einer Reinigungskammer 40, in welche im Ausführungsbeispiel bis zu vier dentaltechnische Geräte oder Instrumente 4 auf Positionierungspunkten, die im Ausführungsbeispiels als Anschlussstutzen 41 ausgebildet sind, aufgesteckt werden. In dem dentaltechnischen Gerät oder Instrument 4 sind Leitungssysteme (in Fig. 2 nicht dargestellt) vorgesehen, durch die beim Betrieb des dentaltechnischen Gerätes oder Instrumentes 4 notwendige Medien, wie beispielsweise Druckluft, Wasser oder auch Desinfektionslösungen für den Mundbereich, in einen Bearbeitungskopf, der am dentaltechnischen Gerät oder Instrument 4 angebracht werden kann, geleitet werden. Eine Aufgabe der gattungsgemäßen Pflegevorrichtung ist es, über dieses Leitungssystem eine Reinigung und Pflege des Gerätes zu erreichen.

Dabei kann zum Einen eine Reinigung des inneren Bereiches des Gerätes oder Instrumentes 4 durchgeführt werden, zum Anderen kann das Leitungssystem auch genutzt werden, um Wartungflüssigkeiten, wie beispielsweise Öle oder sonstige Schmiermittel, in dem Instrument zu verteilen und so dessen dauerhafte Einsatzfähigkeit sicherzustellen. Das Leitungssystem dient daneben auch dazu, eine äußerliche Reinigung des Gerätes oder Instrumentes 4, beispielsweise eine Desinfektion, durchzuführen. Hierbei besteht dann die Möglichkeit, dass in der Reinigungskammer 40 zusätzlich entsprechende Sprühköpfe angeordnet sind, die dann mit entsprechenden Reinigungs- oder Desinfektionsmedien beaufschlagt werden können.

Um die Reinigungskammer 40 beziehungsweise die darin angeordneten Geräte oder Instrumente mit den jeweils vorgesehenen Reinigungs- oder Pflegemedien zu versorgen, sind mehrere Medienleitungen 17a bis 17g als Versorgungsleitungen vorgesehen, die in einer Dosier- und Verteilvorrichtung 18 zusammengeführt sind. Die Medienleitungen 17a bis 17h können dabei auch als Leitungsbündel zu einer Medienleitung zusammengefasst werden. Im Ausführungsbeispiel der Fig. 2 ist die Dosier- und Verteilvorrichtung 18 als gesonderter Block oder Ventilblock ausgeführt, der zwischen den Vorratsgefäßen 12a, 12b für die jeweiligen Reinigungs- und Pflegemedien und der Reinigungkammer 40 angeordnet ist.

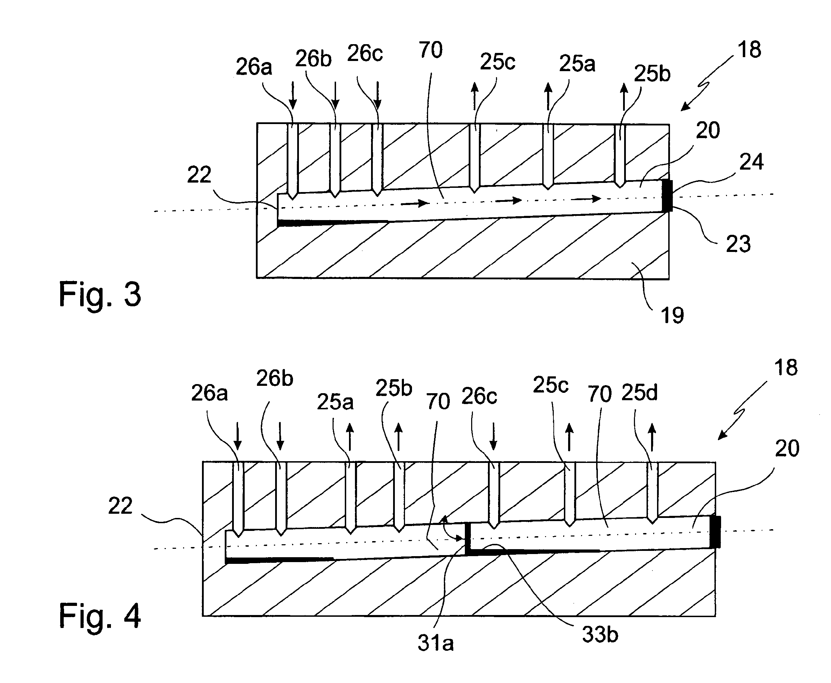

Gestrichelt in der Dosier- und Verteilvorrichtung 18 ist die Aufnahmekammer 70 angedeutet. Wesentlicher Vorzug der Erfindung ist, dass mehrere Medienleitungen 50a, 50b von den Vorgefäßen 12a, 12b in dieser gemeinsamen Aufnahmekammer 70 münden. Dieser Vorzug wird insbesondere in den Details nach Fig. 3, 4 und 5 nochmals deutlich.

Um die Reinigungskammer 40 mit Reinigungs- und Pflegemedien zu versorgen, werden diese in den im Ausführungsbeispiel getrennt voneinander angeordneten Vorratsgefäßen 12a, 12b vorgehalten. In den verschiedenen Vorratsgefäßen 12a, 12b, kann dann jeweils ein gesondertes Reinigungs- beziehungsweise Pflegemedium 16 vorgehalten werden. So kann beispielsweise in dem ersten Vorratsgefäß 12a ein Reinigungs- und Desinfektionsmittel für das Gerät oder Instrument 4 bevorratet werden, während im Vorratsgefäß 12b ein Schmiermittel oder Öl für die Mechanik des Gerätes oder Instrumentes 4 beinhaltet ist. Die Vorratsgefäße 12a, 12b sind über Medienleitungen 50a, 50b mit der Dosier- und Verteilvorrichtung 18 verbunden. Die genaue Ausgestaltung der Dosier- und Verteilvorrichtung 18 kann aus den Figuren 3 bis 5 entnommen werden. Eine entsprechende Beschreibung erfolgt nachfolgend.

Gemäß der Erfindung ist eine Einleitung der Pflege- oder Reinigungsmedien 16 in die Dosier- und Verteilvorrichtung 18 allein durch Schwerkraftbeaufschlagung vorgesehen. Das heißt, in den Medienleitungen 50a, 50b beziehungsweise den Vorratsgefäßen 12a, 12b ist keine weitere Pump- oder Saugvorrichtung vorgesehen, um das Medium 16 in die Dosier- und Verteilvorrichtung 18 zu überführen. Dies setzt jedoch voraus, dass stets ein ausreichender Druckunterschied zwischen den Vorratsgefäßen 12a, 12b und der Dosier- und Verteilvorrichtung 18 herrscht. Um sicherzustellen, dass stets ausreichend Reinigungs- und/oder Pflegemedium 16 in den Vorratsgefäßen 12a, 12b vorhanden ist, weisen diese Drucksensoren 11a, 11b auf, die über Druckleitungen 29a, 29b mit den Vorratsgefäßen 12a, 12b verbunden sind. Die Drucksensoren 11a, 11b wiederum sind mit der Auswert- und Steuereinheit 30 verbunden, in der eine Auswertung der ermittelten Druckwerte erfolgt. Die Druckleitungen 29a, 29b sind am Boden 13 der Vorratsgefäße 12a, 12b angeordnet. Zusätzlich zu der Ermittlung des Füllstandes über den durch den Drucksensor 11a, 11b ermittelten Werte kann an den Vorratsgefäßen 12a, 12b eine Füllstandsanzeige 21 oder entsprechend weitere Sensoren zur Füllstandsermittlung vorgesehen werden. Die Füllstandsanzeige 21 ist im Ausführungsbeispiel als Skala realisiert und dient vor allem dazu, einem Bediener der erfindungsgemäßen Pflegevorrichtung, einen ersten Eindruck über den Füllstand zu vermitteln und auch dafür abschätzen zu können, wie viel an fehlendem Medium 16 in das jeweilige Vorratsgefäß 12a, 12b eingefüllt werden muss, um einerseits eine ausreichende Befüllung des entsprechenden Vorratsgefäßes 12a, 12b durchzuführen und um andererseits eine Überfüllung des Vorratsgefäßes 12a, 12b zu verhindern. Der mit den Drucksensoren 11a, 11b gemessene Druck wird über die Datenleitungen 51a, 51b zur Auswert- und Steuereinheit 30 geleitet. Diese ist dabei beispielsweise Teil einer mehrere Funktionen umfassenden Gerätesteuerung. Die Auswert- und Steuereinheit 30 ermittelt im Ausführungsbeispiel der Fig. 2 aufgrund des durch die Drucksensoren 11a, 11b ermittelten Druckwerte den herrschenden statischen Druck in den Vorratsgefäßen 12a, 12b und leitet daraus das noch zur Verfügung stehende Medienvolumen ab. Dieses kann dann beispielsweise auch über eine Anzeige 32 angezeigt werden. Die Anzeige 32 ist hierzu ebenfalls über eine Datenleitung 51c mit der Auswert- und Steuereinheit 30 verbunden. In der Auswert- und Steuereinheit 30 ist in Fig. 2 gestrichelt eine interne Unterteilung angedeutet. Hierdurch soll zum Ausdruck gebracht werden, dass die Auswert- und Steuereinheit 30 mehrere Teile umfasst, zum Einen eine Auswerteinheit für die ermittelten Druckwerte und zum Anderen eine Steuereinheit, insbesondere für die Dosier- und Verteilvorrichtung 18. Für die Steuerung der Aufgaben beziehungsweise Bestandteile der Dosier- und Verteilvorrichtung 18 ist diese über eine Datenleitung 51d mit der Auswert- und Steuereinheit 30 verbunden. In der letztendlichen Ausführungsform werden die einzelnen Aufgaben der Auswert- und Steuereinheit 30 in einer speicherprogramierbaren Steuerung oder einer mikrokontrollerbasierten Steuerung realisiert. Hierzu wird dann ein entsprechendes Steuerungsprogramm in der Auswert- und Steuereinheit 30 durchlaufen und die vielfältigen Aufgaben der Auswert- und Steuereinheit 30 durch dieses übernommen.

Der Dosier- und Verteilvorrichtung 18 ist eine zusätzliche Medienleitung 50 zugeordnet. Diese dient zur Beaufschlagung der Dosier- und Verteilvorrichtung 18 mit Druckluft und ist entweder mit einem entsprechenden Druckluftreservoir oder einem Kompressor (beides in Fig. 2 nicht dargestellt) verbunden.

Die Ermittlung des in den Vorratsgefäßen 12a, 12b herrschenden Druckes ermöglicht die schwerkraftbeaufschlagte Einleitung der Reinigungs-, Desinfektions- beziehungsweise Pflegemedien 16 in die Dosier- und Verteilvorrichtung 18. Aufgrund der druckabhängigen Ermittlung des Füllstandes in den Vorratsgefäßen 12a, 12b wird sichergestellt, dass stets eine ausreichende Menge an Medien 16 in diesen Vorratsgefäßen 12a, 12b vorhanden ist, um die Einleitung einer ausreichenden Menge an Medium 16 sicherzustellen. Erreicht der ermittelte Füllstand beispielsweise einen Wert für den Mindestfüllstand oder würde bei einer weiteren Entnahme von Medium 16 dieser Mindestfüllstand unterschritten, so kann durch die Auswert- und Steuereinheit eine Blockade der gesamten Pflegevorrichtung durchgeführt werden. Hierdurch wird zum Einen ein Leerlaufen der Vorratsgefäße 12a, 12b und zum Anderen eine Einleitung einer unzureichenden Menge an Medium 16 in die Reinigungskammer 40 verhindert. Dies erweist sich im Interesse einer vollständigen und zufriedenstellenden Reinigung beziehungsweise Pflege der dentaltechnischen Geräte oder Instrumente 4 als besonders vorteilhaft. Wird die Unterschreitung eines Mindestfüllstandes durch die Auswert- und Steuereinheit 30 festgestellt, so besteht die Möglichkeit, über die Anzeige 32 ein entsprechendes Warnsignal (akustisch oder optisch) auszugeben. Dies geschieht beispielsweise gleichzeitig mit oder auch vor der Sperrung der gesamten Pflegevorrichtung. Die Auswert- oder Steuereinheit 30 erfasst auch die Anzahl der in der Reinigungskammer 40 angeordneten Geräte oder Instrumente 4. Aufbauend darauf wird nach Auswahl eines Reinigungs- oder Pflegeprogramms und der dort abgelegten Parameter, die Menge der benötigten Medien 16 ermittelt. Ebenfalls ausgehend von der Anzahl der Geräte oder Instrumente 4 in der Reinigungskammer 40 erfolgt eine Steuerung der Dosier- und Verteilvorrichtung 18. Hierauf wird jedoch im Zusammenhang mit der Beschreibung der Fig. 3 bis 5 weiter eingegangen. Hat nun die Auswerte- und Steuereinheit 30 den Bedarf an Medium 16 sowie die Höhe der notwendigen Druckbeaufschlagung über die Medienleitung 50 für Druckluft ermittelt, wird die Medienmenge mit der in den Vorratsgefäßen 12a, 12b vorhandenen Medienmenge verglichen. Erst wenn festgestellt wird, dass hier ausreichend Medium 16 bevorratet ist, beginnt die Pflegevorrichtung mit der Reinigung und Pflege beziehungsweise Wartung der Geräte oder Instrumente 4 in der Reinigungskammer 40. Hierbei wird dann ein in der Auswert- und Steuereinheit 30 abgelegtes Reinigungsprogramm durchlaufen, das gegebenenfalls mehrere Reinigungs-, Pflege- und Druckbeaufschlagungschritte umfasst. Die Druckbeaufschlagung der Dosier- und Verteilvorrichtung 18 dient dabei auch dazu, dass schwerkraftbeaufschlagt in die Dosier- und Verteilvorrichtung 18 eingeleitete Medium 16 in die Reinigungskammer 40 beziehungsweise die darin angeordneten Instrumente und Geräte 4 einzutragen. Daneben kann über die Druckluft auch eine Vorreinigung beziehungsweise Trocknung der Geräte oder Instrumente 4 durchgeführt werden. Ebenfalls möglich ist hierdurch eine verbesserte Verteilung von Pflegemedium 16 in dem Gerät oder Instrument 4.

Fig. 3 zeigt eine bevorzugte Ausführungsform der Dosier- und Verteilvorrichtung 18 in einer seitlichen Schnittdarstellung. Die Dosier- und Verteilvorrichtung 18 verfügt über einen Grundkörper 19, in den eine Transportleitung 20 eingebracht ist. Diese Transportleitung 20 ist an ihrem ersten, in Fig. 3 linken Ende 22 abgeschlossen. An ihrem zweiten, in Fig. 3 rechten Ende 23 ist die Transportleitung 20 durch eine abnehmbare Verschlusskappe 24 verschlossen, die einen flüssigkeits- und druckdichten Abschluss der Transportleitung 20 erlaubt, im Falle, dass die Dosier- und Verteilvorrichtung erweitert werden soll, jedoch die Anbindung weiterer Dosier- und Verteilvorrichtungen 18 ermöglicht. Die Transportleitung 20 ist im Grundkörper 19 gegenüber der Horizontalen geneigt angeordnet. Hieraus ergibt sich dann, dass sich im Bereich des ersten, linken Endes 22 der tiefste Punkt der Transportleitung 20 befindet. Erfolgt nun die Einleitung eines Mediums 16 in die Dosier- und Verteilvorrichtung 18, so wird sich dieses Medium 16 zunächst im Bereich des linken, ersten Endes 22, das den tiefsten Punkt der Transportleitung 20 darstellt, sammeln und kann von hier aus dann nach Druckbeaufschlagung in der Transportleitung 20 zu den Auslässen 25a, 25b, 25c geführt werden. Zu diesem Zweck weist die Dosier- und Verteilvorrichtung 18 einen Einlass 26a auf, über den beispielsweise ein Transportmedium wie Druckluft in die Transportleitung 20 eingeleitet werden kann. Der Einlass 26a kann hierzu beispielsweise mit einer Druckluftleitung oder einem Kompressor verbunden werden. In Strömungsrichtung des Mediums 16, dem ersten Einlass 26a nachgeordnet, befinden sich zwei weitere Einlässe 26b, 26c, über die ein Eintrag von Medien 16 in die Dosier- und Verteilvorrichtung 18 durchgeführt werden kann. Bei den hier eingetragenen Medien 16 kann es sich zum einen um Reinigungs- und/oder Desinfektionsmittel und zum anderen um Wartungs- beziehungsweise Pflegemittel, wie beispielsweise Schmierstoffe oder Öle handeln. Denkbar ist aber auch die Zuführung von weiterem Reinigungs- oder Pflegemittel. Diese werden über die Dosier- und Verteilvorrichtung 18 der Reinigungskammer 40 (vergleiche Fig. 2) zugeleitet. Die über die Einlässe 26b, 26c eingetragenen Medien 16 sammeln sich in dem unteren linken Bereich des ersten Endes 22 der Dosier- und Verteilvorrichtung 18. Von hier aus wird das gesammelte Medium 16 durch Einlassen von im Ausführungsbeispiel Druckluft durch den Einlass 26a in der Transportleitung 20 verfrachtet und zu den Auslässen 25a, 25b und 25c transportiert. Diese Auslässe 25a, 25b, 25c sind über Medienleitungen (nicht dargestellt) mit einer Reinigungskammer 40 verbunden, in der dentaltechnische Geräte oder Instrumente 4 angeordnet sind. In Fig. 3 dargestellt ist eine besonders einfache Ausführungsform der Dosier- und Verteilvorrichtung 18 für die erfindungsgemäße Pflegevorrichtung. Hierbei erfolgt der Austrag des Mediums 16 aus jeweils einem, einem jeden Instrument 4 zugeordneten Auslass 25a, 25b, 25c. Bezüglich der Medien 16, die in die Dosier- und Verteilvorrichtung eingeleitet werden besteht die Möglichkeit, hier wenigstens zwei verschiedene Medien 16 in die Dosier- und Verteilvorrichtung 18 über zwei getrennte Einlässe 26b, 26c zuzuführen. So kann beispielsweise über einen ersten Einlass 26b ein Reinigungs- und/oder Desinfektionsmedium in die Dosier- und Verteilvorrichtung 18 eingebracht werden, während über einen anderen Einlass 26c ein Pflegemedium, beispielsweise Schmieröl oder ein sonstiger Pflegestoff zugeführt wird.

Die Fig. 4 zeigt eine weitere mögliche Ausführungsform der Dosier- und Verteilvorrichtung 18, die in der erfindungsgemäßen Pflegevorrichtung verwendbar ist. Diese ist gegenüber der in Fig. 3 dargestellten Vorrichtung dahingehend weitergebildet, dass hier ein erster Einlass 26a für die Druckbeaufschlagung der Medien im linken Bereich, das heißt im Bereich des ersten Endes 22 der Dosier- und Verteilvorrichtung 18 vorgesehen ist, über den eine Beaufschlagung der gesamten Dosier- und Verteilvorrichtung 18 erfolgt. Dem ersten Einlass 26a nachgeordnet ist ein weiterer Einlass 26b für ein erstes Medium 16, bei dem es sich beispielsweise um ein Reinigungs- oder Desinfektionsmedium handeln kann, vorgesehen. Dem Medieneinlass 26b nachgeordnet finden sich zwei Auslässe 25a, 25b, die mit einer Reinigungskammer 40 beziehungsweise mit dem Leitungssystem eines in der Reinigungskammer 40 angeordneten dentaltechnischen Instrumentes 4 verbunden werden können. Über diese Auslässe 25a, 25b kann somit eine Beaufschlagung des Instrumentes 4 mit dem ersten, über den Einlass 26b in die Dosier- und Verteilvorrichtung 18 eingebrachten Medium 16 erfolgen. In der Fließ- beziehungsweise Transportrichtung des Mediums 16 in der Dosier- und Verteilvorrichtung 18 angeordnet befindet sich eine Sperrvorrichtung 31a, mit der sich die Transportleitung 20 in zwei Bereiche unterteilen läßt. Durch die Sperrvorrichtung 31a kann hier auch ein weiteres Zwischendepot 33b für ein weiteres Medium 16 gebildet werden. Dies ergibt sich dadurch, dass die Transportleitung 20 in der Dosier- und Verteilvorrichtung 18 gegenüber der Horizontalen geneigt angeordnet ist, und sich hier somit ein bei Verschließen der Sperrvorrichtung 31a ein tiefster Punkt des Transportleitungsbereichs bildet, der als Sammelstelle für das Medium 16 dient. Der Sperrvorrichtung 31a unmittelbar nachgeordnet befindet sich ein weiterer Einlass 26c für ein Medium 16. Über diesen kann ein weiteres, vom ersten unterschiedliches Medium 16 in die Dosier- und Verteilvorrichtung 18 eingebracht werden. Hierbei kann es sich beispielsweise um ein Pflegemedium handeln. Aufgrund der Sperrvorrichtung 31a besteht die Möglichkeit, die Dosier- und Verteilvorrichtung 18 dahingehend abzutrennen, dass lediglich eine Zuleitung von Medium 16 zu den an den Auslässen 25c, 25d angeordneten Geräten oder Instrumenten 4 erfolgt. Dieses Ausführungsform der Dosier- und Verteilvorrichtung 18 hat den Vorteil, dass bei geöffneter Sperrvorrichtung 31a ein Eintrag von einem ersten Medium 16 in alle Instrumente 4 erfolgen kann, die über entsprechende Leitungen mit der Dosier- und Verteilvorrichtung 18 verbunden sind. Dies kann beispielsweise dazu genutzt werden, um ein Reinigungs- und Desinfektionsmedium, das für alle Instrumente 4 geeignet ist, in diese Instrumente 4 einzubringen. Ist des Weiteren vorgesehen, dass eine Pflege, beispielsweise eine Schmierung von nur einem Teil der Instrumente 4 erfolgt, beispielsweise von solchen Instrumenten 4, die mit drehenden Teilen versehen sind, so kann hier eine Absperrung der Transportleitung 20 erfolgen und ein Eintrag des entsprechenden Pflegemediums nur in zwei der vier an die Dosier- und Verteilvorrichtung 18 maximal ankoppelbaren Instrumente 4 erfolgt.

Neben der Beaufschlagung der Dosier- und Verteilvorrichtung 18 mit Druckluft über den Einlass 26a, besteht selbstverständlich auch die Möglichkeit, reinigungskammerseitig einen Unterdruck anzulegen, so dass das entsprechende Medium 16 in die in der Reinigungskammer 40 angeordneten Instrumente 4 eingesaugt wird. Hierzu empfiehlt es sich dann, im Augenblick der Unterdruckbeaufschlagung, den Einlass 26a zu öffnen, um hier einen Druckausgleich zu schaffen, der das Medium 16 letztendlich in die Instrumente 4 einträgt. Auch die Dosier- und Verteilvorrichtung 18 der Fig. 4 kann durch weitere gleichartige Module erweitert werden. Unter einem Modul ist hierbei eine Einheit beziehungsweise eine Dosier- und Verteilvorrichtung 18 zu verstehen, die wenigstens einen Einlass 26a für die Druckbeaufschlagung sowie einen weiteren Einlass 26b für den Eintrag von Medium 16 sowie einen oder zwei Auslässe 25c, 25d für den Austrag des Mediums 16 aus der Dosier- und Verteilvorrichtung 18 und dessen Eintrag in Reinigungskammer 40 aufweist. Durch diese mögliche Erweiterung der Dosier- und Verteilvorrichtung 18 kann diese flexibel eingesetzt werden, wenn beispielsweise ein höherer Reinigungsbedarf besteht oder eine Erweiterung bestehender Pflegevorrichtungen geplant ist.

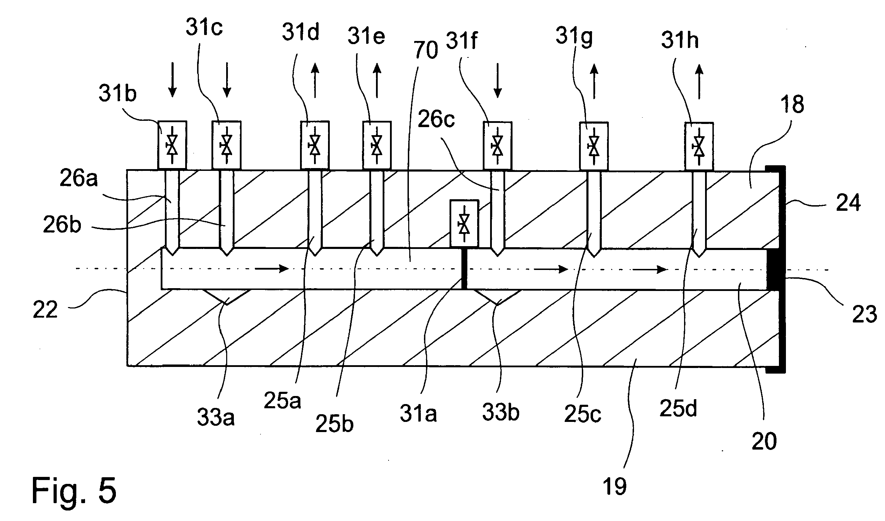

Fig. 5 zeigt eine weitere mögliche Ausführungsform der Dosier- und Verteilvorrichtung 18. Dieses weist eine horizontal in der Dosier- und Verteilvorrichtung 18, die als Block ausgebildet ist, verlaufende Transportleitung 20 auf. Diese Transportleitung 20 ist an einem ersten Ende 22 der Dosier- und Verteilvorrichtung 18 geschlossen, während die Transportleitung 20 an dem gegenüberliegenden zweiten Ende 23 der Dosier- und Verteilvorrichtung 18 ein offenes Ende aufweist, das im Ausführungsbeispiel über eine Verschlusskappe 24 verschlossen ist. Diese Verschlusskappe 24 ist flüssigkeits- und druckdicht in die Dosier- und Verteilvorrichtung 18 eingesetzt und kann, beispielsweise um die Dosier- und Verteilvorrichtung 18 zu erweitern, von dieser abgenommen werden. Die Transportleitung 20 ist in den die Dosier- und Verteilvorrichtung 18 bildenden Grundkörper 19 im Zuge einer Bohrung beziehungsweise Ausfräsung eingebracht.

In der Transportleitung 20 sind die Zwischendepots 33a und 33b angeordnet. Diese werden im Zuge der Bildung der Transportleitung 20 in den Grundkörper 19 der Dosier- und Verteilvorrichtung 18 eingefräst und sind im Ausführungsbeispiel als entsprechende Vertiefungen ausgebildet. Daneben besteht selbstverständlich auch die Möglichkeit, in Abhängigkeit von der letztendlichen Ausgestaltung der Dosier- und Verteilvorrichtung 18, hier quer zur Transportrichtung des Mediums 16 ausgerichtete Rinnen anzuordnen. Auch müssen die Zwischendepots 33a und 33b nicht zwingend mit einem spitz zulaufenden Boden ausgebildet werden, wie dies in Fig. 5 dargestellt ist. Es besteht hier auch die Möglichkeit, diesen beispielsweise nach Art eines Topfes auszubilden. Diese sind unmittelbar Einlässen 26b, 26c, für Medien 16, beispielsweise für ein Reinigungsmedium im Fall von Einlass 26b und ein Pflegemedium im Fall von Einlass 26c zugeordnet. Über die Einlässe 26b und 26c erfolgt dann ein ausschließlich schwerkraftbeaufschlagtes Einbringen von Medien 16 aus einem Vorratsgefäß 12a, 12b (vergleiche Fig. 2) in die Dosier- und Verteilvorrichtung 18. Die Dosier- und Verteilvorrichtung 18 umfasst weiterhin eine Sperrvorrichtung 31a, um die Transportleitung 20 in zwei Bereiche zu unterteilen. Der erste Einlass 26b für das Medium 16 ist dabei in Fließrichtung des Mediums 16 in der Transportleitung 20 vor der Sperrvorrichtung 31a angeordnet, während der zweite Einlass 26c sowie das diesem Einlass 26c zugeordnete Zwischendepot 33b nach dieser Sperrvorrichtung 31a angeordnet ist. Die Verwendung einer Sperrvorrichtung 31a in der Transportleitung 20 bietet die Möglichkeit, dass hier eine gesteuerte Beaufschlagung der Instrumente 4 mit unterschiedlichen Medien 16 erfolgen kann. So besteht beispielsweise die Möglichkeit, dass über den ersten Einlass 26b für ein erstes Medium 16 beispielsweise ein Pflegemedium zugeführt wird, und bei geöffneter Sperrvorrichtung 31a ein Eintrag dieses Pflegemediums in alle an den Auslässen 25a, 25b, 25c und 25d angeordneten Instrumenten 4 erfolgt. Wird die Sperrvorrichtung 31a geschlossen, so erfolgt nur ein Eintrag von Medium 16 in die an den Auslässen 25a, 25b angeordneten Instrumente 4. Dies erweist sich beispielsweise dann als vorteilhaft, wenn nur ein Eintrag von Medium 16 in die an den ersten Auslässen 25a, 25b angeordneten Instrumente 4 vorgesehen ist.

Die in der Fig. 5 dargestellte Dosier- und Verteilvorrichtung 18 ermöglicht eine hochgenaue Dosierung und Zuführung von Medien 16 zu einer Reinigungskammer 40. Dies ist dadurch bedingt, dass den Einlässen 26b, 26c beziehungsweise Auslässen 25a, 25b. 25c, 25d jeweils gesonderte und gesondert ansteuerbare Sperrvorrichtungen 31b bis 31h zugeordnet sind. Diese können über eine Auswert- und Steuereinheit 30 auf die jeweils zu reinigende Instrumente 4 abgestimmt angesteuert werden. Ebenfalls möglich ist, diese einzelnen Sperrvorrichtungen 31b bis 31h nur dann anzusteuern, wenn einerseits ein entsprechendes Medium 16 in die Dosier- und Verteilvorrichtung 18 eingeführt werden soll und andererseits auch ein dentaltechnisches Instrument 4 in der Reinigungskammer 40 angeordnet ist, das mit dem entsprechenden Medium 16 beaufschlagt werden muss. Über eine entsprechend geschickte Programmierung des Steuerprogramms kann somit eine Vielzahl von Reinigungsschritten und eine Vielzahl von unterschiedlichen Instrumenten 4 gleichzeitig in der Reinigungskammer 40 mit unterschiedlichen Reinigungs- und Pflegeprogrammen bearbeitet werden, ohne dass nach jedem Reinigungsprogramm ein Austausch der Instrumente 4 durchgeführt werden muss. Dies führt zu einer Einsparung von Kosten einerseits und andererseits zu einem Zeitgewinn. Darüber hinaus kann auch durch die Auswert- und Steuereinheit 30 eine Erfassung der einzelnen Reinigungsschritte erfolgen, so dass hier eine lückenlose Dokumentation der Reinigungs- und Pflegetätigkeit durchgeführt werden kann. Die Steuerung der Sperrvorrichtungen 31b bis 31h, die unter anderem als Magnetventil ausgeführt werden können, erfolgt ebenfalls über das Steuerprogramm, das in der Auswert- und Steuereinheit 30 abgelegt ist. Hierbei kann auch eine Ansteuerung der entsprechenden Sperrvorrichtungen 31c und 31f für die Einlässe 26b und 26c, die mit den Vorratsgefäßen 12a, 12b für die zu verwendenden Medien 16 verbunden sind, in Abhängigkeit von dem Füllstand in den entsprechenden Vorratsgefäßen 12a, 12b erfolgen. Da hier eine allein schwerkraftbeaufschlagte Zuführung von Medium 16 erfolgt, ist diese abhängig vom jeweils in den Vorratsgefäßen 12a, 12b herrschenden Füllständen. Hierauf muss somit die Öffnungsdauer der Sperrvorrichtungen 31c und 31f, die den entsprechenden Einlässen 26b, 26c zugeordnet sind, abgestimmt werden, um stets die gleiche Menge beziehungsweise eine ausreichende Menge an Medium 16 in die Dosier- und Verteilvorrichtung 18 einfüllen zu können. Hierzu ist eine Verbindung zwischen den entsprechenden Sensoren 11a, 11b für den Füllstand der Steuer- und Auswerteinheit 30 und den entsprechenden Sperrvorrichtungen 31c und 31f vorgesehen. Ebenfalls über die Steuer- und Auswerteinheit 30 wird eine Öffnung beziehungsweise ein Verschluss der Sperrvorrichtungen 31d, 31e, 31g, 31h, die den Auslässen 25a, 25b, 25c, 25d zu den Geräten 4 zugeordnet sind, bewirkt.

In den Fig. 3, 4 und 5 ist mit 70 die gemeinsame Aufnahmekammer 70 für die verschiedenen Medienleitungen beziehungsweise den hier dargestellten Einlässen 26b, c gezeigt. Es ist klar, dass durch die gemeinsame Ausnutzung der Aufnahmekammer 70 für verschiedene Einlässe, die durch Medienleitungen mit dem jeweiligen Vorratsbehälter 12a, 12b verbunden sind, der konstruktive Aufwand erheblich reduziert ist.

## Patentansprüche

1. Pflegevorrichtung für wenigstens ein dentaltechnisches Gerät (4), umfassend wenigstens ein Vorratsgefäß (12) für ein Fluid oder eine Flüssigkeit (16), insbesondere ein Reinigungs-, Desinfektions- oder Pflegemedium, wenigstens eine Reinigungskammer (40) zur Anordnung des dentaltechnischen Gerätes (4) und wenigstens eine das Vorratsgefäß (12) und die Reinigungskammer (40) verbindende Medienleitung (17a,17b), wobei eine Messeinrichtung (10) zur Ermittlung der im Vorratsgefäß (12) vorhandenen Menge und/oder des Füllstandes des Fluids oder der Flüssigkeit (16) vorgesehen ist und die Messeinrichtung (10) wenigstens einen, insbesondere datentechnisch auslesbaren Sensor (11) aufweist, **dadurch gekennzeichnet, dass** der Sensor (11) als Drucksensor ausgebildet ist.

2. Pflegevorrichtung nach Anspruch 1, **dadurch gekennzeichnet, dass** über den Sensor (11) eine Änderung des im Vorratsgefäß (12) herrschenden statischen Druckes und/oder eine bei der Entnahme des Fluids oder der Flüssigkeit (16) auftretende Druckänderung zur Ableitung des Füllstandes beziehungsweise einer Änderung des Füllstandes erfassbar ist und/oder der Drucksensor (11) über eine Druckleitung (29) mit dem Vorratsgefäß (12), insbesondere im Bodenbereich (13) des Vorratsgefäßes (12) verbunden ist.

3. Pflegevorrichtung nach einem der vorhergehenden Ansprüche, **dadurch gekennzeichnet, dass** eine Auswerteinheit (30) für die durch den Sensor (11) erfassten Werte für den statischen Druck und/oder die Änderung des Druckes vorgesehen ist und insbesondere eine optische oder akustische Anzeige (32) vorgesehen ist, wobei die Anzeige (32) mit der Auswerteinheit (30) und/oder einer Steuereinheit (31) verbunden ist.

4. Pflegevorrichtung nach einem der vorhergehenden Ansprüche, **dadurch gekennzeichnet, dass** eine kontinuierliche Erfassung des statischen Druckes vorgesehen ist beziehungsweise eine kontinuierliche Erfassung der Druckänderung, insbesondere während der Entnahme des Fluids oder der Flüssigkeit (16), vorgesehen ist.

5. Pflegevorrichtung nach einem der vorhergehenden Ansprüche, **dadurch gekennzeichnet, dass** eine Steuereinheit (31) für die Entnahmemenge und/oder Entnahmedauer des Fluids oder der Flüssigkeit (16) aus dem Vorratsgefäß (12) vorgesehen ist, welche mit der Auswerteinheit (30) verbunden ist und auf die Dosiervorrichtung (18) wirkt, wobei die Steuerung der Entnahmemenge und/oder Entnahmedauer anhand des ermittelten Füllstandes und/oder des statischen Druckes und/oder des Verlaufs der Druckänderung erfolgt.

6. Pflegevorrichtung nach einem der vorhergehenden Ansprüche, **dadurch gekennzeichnet, dass** eine Speichereinheit (33) zur Erfassung und Speicherung der Betriebszeit, der Betriebsdauer, der Medienentnahmezeit, der Medienentnahmemenge, der Medienentnahmedauer, der erfassten Druckwerte des Füllstandes im Vorratsgefäß und/oder der Medienentnahmemenge aus dem Vorratsgefäß vor und nach der Medienentnahme, die Unterschreitung eines Mindestwertes für den Füllstand, des Zeitpunkts der Sperrung des Auslasses und/oder der Dosiervorrichtung und/oder der Entnahmegeschwindigkeit vorgesehen ist.

7. Pflegevorrichtung, nach einem der vorhergehenden Ansprüche, **gekennzeichnet durch**
- wenigstens zwei Vorratsgefäße für ein Fluid oder eine Flüssigkeit, insbesondere ein Desinfektions-, Reinigungs- oder Pflegemedium,
- jeweils eine das Vorratsgefäß und die Reinigungskammer verbindende Medienleitung, und
- eine in den Medienleitungen (50a,b, 17a-h) angeordnete Dosier- und Verteilvorrichtung (18) für das Reinigungs- oder Pflegemedium
wobei die Dosier- und Verteilvorrichtung (18) eine für die an der Dosier- und Verteilvorrichtung (18) angeschlossenen, von den Vorratsgefäßen herführenden Medienleitungen gemeinsame Aufnahmekammer (70) aufweist, und die Aufnahmekammer (70) als Transportleitung Teil der Medienleitung ist und eine schwerkraftbeaufschlagte Überführung des Mediums (16) aus dem Vorratsgefäß (12a, 12b) in die Aufnahmekammer der Dosier- und Verteilvorrichtung (18) vorgesehen ist.

8. Pflegevorrichtung nach Anspruch 7, **dadurch gekennzeichnet, dass** ein Eintrag des Fluids oder der Flüssigkeit (16) in die Reinigungskammer (40) nach Druckbeaufschlagung, insbesondere nach Über- oder Unterdruck oder nach Druckluftbeaufschlagung, vorgesehen ist, wobei die Druckbeaufschlagung, insbesondere in der Dosier- und Verteilvorrichtung (18) erfolgt, insbesondere wobei eine Regelungsvorrichtung für die Höhe der Druckbeaufschlagung vorgesehen ist und/oder die Dosier- und Verteilvorrichtung (18) wenigstens umfasst:
- eine Transportleitung (20) für das Reinigungs- oder Pflegemedium (16),
- wenigstens ein in der Transportleitung (20) angeordnetes oder der Transportleitung (20) zugeordnetes Zwischendepot (33a,b) für das Pflegemedium (16), wobei der Transportleitung (20) zugeordnet, insbesondere in die Transportleitung (20) mündend, wenigstens
- ein Einlass (26a) für eine Druckbeaufschlagung und/oder ein Transportmedium
- ein Einlass (26b, 26c) für das Pflegemedium (16), und
- mindestens ein Auslass (25a,b,c,d) für das Medium (16) zugeordnet ist, und der Einlass (26b,26c) für das Fluid oder die Flüssigkeit (16) mit dem Vorratsgefäß (12a,12b) und der Auslass (25a,b,c,d) mit der Reinigungskammer (40) über die Medienleitung (50a,b, 17a-h) verbunden ist.

9. Pflegevorrichtung nach einem der vorhergehenden Ansprüche 7 und 8, **dadurch gekennzeichnet, dass** wenigstens eine erste Sperrvorrichtung (31a), insbesondere ein Elektromagnetventil und/oder eine Klappe oder ein Schieber, zur Unterteilung der Transportleitung (20) vorgesehen ist und/oder jeweils eine dem Einlass (26a) für die Druckbeaufschlagung bzw. das Transportmedium und/oder dem Einlass (26b,c) und/oder dem Auslass (25a,b,c,d) für das Pflegemedium (16) zugeordnete weitere Sperrvorrichtung (31b-h), insbesondere ein weiteres Elektromagnetventil, vorgesehen ist.

10. Pflegevorrichtung nach einem der vorhergehenden Ansprüche 7 bis 9, **dadurch gekennzeichnet, dass** eine Auswert- und Steuereinheit (30) für die Sperrvorrichtung(en) (31a-h) insbesondere mit wenigstens einem hinterlegten Steuerungsprogramm vorgesehen ist, wobei insbesondere eine Steuerung der Öffnungs- und Verschlusszeitpunkte und/oder - abfolge und/oder der Öffnungs- und Verschlussdauer der Sperrvorrichtung(en) (31a-h) erfolgt und/oder die Betätigung der dem Einlass (26b,c) für das Fluid oder die Flüssigkeit (16) zugeordneten Sperrvorrichtung (31c,g) anhand von für den Füllstand des Pflegemediums (16) im Vorratsgefäß (12a,12b) ermittelten Füllstandswerten, insbesondere basierend auf für den Füllstand ermittelten Druckwerten, erfolgt.

11. Pflegevorrichtung nach einem der vorhergehenden Ansprüche 8 bis 10, **dadurch gekennzeichnet, dass** das Zwischendepot (33a,b) im Bereich des Einlasses (26b,c) für das Pflegemedium (16) angeordnet ist und/oder das Zwischendepot (33a,b) als Becken oder Rinne in der Transportleitung (20) und/oder als an einem Ende (22,23) der Transportleitung (20) angeordnete Aufweitung ausgebildet ist und/oder die Transportleitung (20) in der Dosier- und Verteilvorrichtung (18) insbesondere gegenüber der Horizontalen geneigt verlaufend eingebracht ist und ein tieferliegendes Ende (22,23) der Transportleitung (20) als Zwischendepot (33a,b) dient.

12. Pflegevorrichtung nach einem der vorhergehenden Ansprüche 7 bis 11, **dadurch gekennzeichnet, dass** in der Dosier- und Verteilvorrichtung (18) einem ersten oder zweiten Ende (22,23) zugeordnet der Einlass (26a) für die Druckbeaufschlagung und/oder das Transportmedium vorgesehen ist, während in Transportrichtung des Pflegemediums (16) dem Einlass (26a) nachgeordnet der Einlass (26b,c) für das Fluid oder die Flüssigkeit (16) sowie der Auslass (25a,b,c,d) für das Fluid oder die Flüssigkeit (16) vorgesehen ist und/oder die Dosier- und Verteilvorrichtung (18) aus wenigstens einem Modul aufgebaut ist und das Modul wenigstens einen Abschnitt der Transportleitung (20), wenigstens ein Zwischendepot (33a,b), wenigstens einen Einlass (26a) für die Druckbeaufschlagung bzw. das Transportmedium, wenigstens einen Einlass (26b,c) und einen Auslass (25a,b,c,d) für das Fluid oder die Flüssigkeit (16) aufweist, insbesondere wobei die Module frei kombinierbar ausgebildet sind, wobei insbesondere in dem Modul jeweils eine Sperrvorrichtung (31b) für die Transportleitung (20) und/oder weitere Sperrvorrichtungen (31c-h) für den Einlass (26a) für die Druckbeaufschlagung beziehungsweise das Transportmedium und/oder den Einlass (26b,c) und/oder den Auslass (25a,b,c,d) für das Fluid oder die Flüssigkeit (16) vorgesehen sind.

13. Verfahren zur Überwachung des Füllstandes eines Mediums, insbesondere eines Reinigungs-, Desinfektions- oder Pflegemediums in einem Vorratsgefäß einer Pflegevorrichtung für wenigstens ein dentaltechnisches Gerät, nach einem der vorhergehenden Ansprüche, umfassend die Schritte:
- sensorgestüzte Erfassung eines in dem Vorratsgefäß vor einer Medienentnahme herrschenden statischen Druckes,
- Auswertung der erfassten Werte und
- Ableitung eines Füllstandes des Mediums in dem Vor ratsgefäß anhand der erfassten Werte.

14. Verfahren nach Anspruch 13, **dadurch gekennzeichnet, dass** die Auswertung in einer Auswerteinheit (30) erfolgt, wobei anhand der durch den Sensor (11) erfassten Werte, insbesondere anhand der Werte eines statischen Druckes und/oder einer Druckänderung eine Berechnung der Medienmenge bzw. des Füllstandes erfolgt und/oder nach der Bestimmung des Füllstandes der Füllstand mit einem definierten Mindestwert verglichen wird und/oder die dem Füllstand entsprechende Füllstandsinformation einer Anzeige zugeleitet wird.

15. Verfahren nach einem der Ansprüche 13 oder 14, **dadurch gekennzeichnet, dass** eine Bestimmung der Entnahmemenge und/oder der Entnahmegeschwindigkeit des Mediums anhand des errechneten Füllstandes und/oder der Änderung des Druckes während oder nach der Medienentnahme erfolgt und/oder in Abhängigkeit des Füllstandes die Dosiervorrichtung gesteuert wird, insbesondere bei geringem Füllstand die Entnahmedauer des Mediums aus dem Vorratsgefäß erhöht wird.

16. Verfahren nach einem der Ansprüche 13 bis 15, **dadurch gekennzeichnet, dass** die Unterschreitung eines Mindestwertes für den Füllstand durch kontinuierlichen Vergleich der während der Entnahme errechneten oder erfassten Füllstands- und/oder Druckwerten und/oder den nach der Entnahme ermittelten statischen Druckwerten mit einem definierten entsprechenden Wert für den Mindestwert für den Füllstand festgestellt wird und/oder bei Unterschreitung eines Mindestwertes für den Füllstand die Ausgabe eines insbesondere optischen oder akustischen Signals erfolgt und/oder eine Sperrung eines insbesondere dem Vorratsgefäß und/oder einer Medienleitung zugeordneten Auslasses und/oder der Dosiervorrichtung erfolgt.

## Claims

1. Cleaning device for at least one dental instrument (4), comprising at least one storage container (12) for a fluid or a liquid (16), in particular a cleaning, disinfection, or care medium, at least one cleaning chamber (40) for arranging the dental instrument (4), and at least one media line (17a, 17b) connecting the storage container (12) and the cleaning chamber (40), wherein a measuring device (10) is provided for determining the amount and/or the filling level of the fluid or the liquid (16) in the storage container (12), and the measuring device (10) has at least one sensor the data of which can be read out, **characterized in that** the sensor (11) is configured as pressure sensor.

2. Cleaning device according to claim 1, **characterized in that** via the sensor (11) a change of the static pressure in the storage container (12) and/or a change of pressure occurring during removing the fluid or the liquid (16) for deriving the filling level and/or a change of the filling level can be recorded, and/or the pressure sensor (11) is connected to the storage container (12), in particular in the bottom area (13) of the storage container (12), via a pressure line (29).

3. Cleaning device according to one of the preceding claims, **characterized in that** an evaluation unit (30) for the values for the static pressure recorded by the sensor (11) and/or the change of the pressure is provided, and in particular an optical or acoustic signal (32) is provided, wherein the signal (32) is connected with the evaluation unit (30) and/or a control unit (31).

4. Cleaning device according to one of the preceding claims, **characterized in that** a continuous recording of the static pressure is provided, or a continuous recording of the pressure change, in particular during the removal of the fluid or the liquid (16) is provided.

5. Cleaning device according to one of the preceding claims, **characterized in that** a control unit (31) is provided for the removed amount and/or removal period of the fluid or the liquid (16) from the storage container (12), the control unit (31) being connected with the evaluation unit (30) and acting on the dosage device (18), wherein the control of the removed amount and/or removal period is performed by means of the determined filling level and/or the static pressure and/or the progress of the pressure change.

6. Cleaning device according to one of the preceding claims, **characterized in that** a storage unit (33) is provided for recording and storing the operating time, operating period, the time for removing the medium, amount of the removed medium, the period for removing the medium, the recorded pressure values of the filling level in the storage container, and/or the amount of the removed medium from the storage container before and after the removal of medium, the shortfall of a minimum value for the filling level, the time of locking the outlet and/or the dosage device and/or the removal speed.

7. Cleaning device according to one of the preceding claims, **characterized by**
- at least two storage containers for a fluid or liquid, in particular for disinfection, cleaning or care medium,
- one media line each connecting the storage container and the cleaning chamber, and
- a dosage and distributing device (18) arranged in the media lines (50a, b, 17 a - h) for the cleaning or care medium,
wherein the dosage and distributing device (18) has a holding chamber (70) common for the media lines connected to the dosage and distributing device (18), coming from the storage containers, and the holding chamber (70) as a transport line is part of the media line, and a transport of the medium (16) impinged by gravity from the storage container (12a, 12b) in the holding chamber of the dosage and distributing device (18) is provided.

8. Cleaning device according to claim 7, **characterized in that** an import of the fluid or the liquid (16) in the cleaning chamber (40) after pressurizing, in particular after positive or negative pressure or pressurizing by compressed air, is provided, wherein the pressurizing is performed, in particular in the dosage and distributing device (18), in particular wherein a regulating device is provided for the level of pressurizing, and/or the dosage and distributing device (18) comprises at least:
- a transport line (20) for the cleaning or care medium (16),
- at least one interim storage (33a, b) for the care medium (16) arranged in the transport line (20) or associated with the transport line (20), wherein associated with the transport line (20), in particular running in the transport line (20) at least
- one inlet (26a) for pressurizing and/or a transport medium,
- one inlet (26b, 26c) for the care medium (16), and
- at least one outlet (25a, b, c, d) for the medium (16) is associated, and the inlet (26b, 26c) for the fluid or the liquid (16) is connected with the storage container (12a, 12b), and the outlet (25a, b, c, d) is connected with the cleaning chamber (40) via the media line (50a, b, 17a - h).

9. Cleaning device according to one of the preceding claims 7 and 8, **characterized in that** at least a first locking device (31a), in particular a solenoid valve and/or flap or slider is provided for sub-dividing the transport line (20), and/or one other locking device (31b - h) each, in particular another solenoid valve is provided associated with the inlet (26a) for pressurizing or the transport medium, and/or the inlet (26b, c) and/or the outlet (25a, b, c, d) for the care medium (16).

10. Cleaning device according to one of the preceding claims 7 to 9, **characterized in that** an evaluation and control unit (30) for the locking device (s) (31a - h), in particular with at least one stored control program is provided, wherein in particular a control of the opening and locking times and/or succession and/or the opening and locking period of the locking device(s) (31a - h) is carried out, and/or activating of the locking device (31c, g) associated with the inlet (26b, c) for the fluid or the liquid (16) is performed by means of the filling level values determined for the filling level of the care medium (16) in the storage container (12a, 12b), in particular based on the pressure values determined for the filling level.

11. Cleaning device according to one of the preceding claims 8 to 10, **characterized in that** the interim storage (33a, b) is arranged in the area of the inlet (26b, c) for the care medium (16), and/or the interim storage (33a, b) is configured as basin or groove in the transport line (20) and/or as widening arranged at an end (22, 23) of the transport line (20), and/or the transport line (20) is put in the dosage and distributing device (18), in particular running inclined with reference to the horizontal line, and an end (22, 23) of the transport line (20) arranged deeper serves as interim storage (33a, b).

12. Cleaning device according to one of the preceding claims 7 to 11, **characterized in that** in the dosage and distributing device (18), associated with a first or second end (22, 23), the inlet (26a) for the pressurizing and/or the transport medium is provided, while in transport direction of the care medium (16) downstream of the inlet (26a) the inlet (26b, c) for the fluid or the liquid (16) as well as the outlet (25a, b, c, d) for the fluid or the liquid (16) is provided, and/or the dosage and distributing device (18) is formed of at least one module, and the module has at least a section of the transport line (20), at least one interim storage (33a, b), at least one inlet (26a) for pressurizing or the transport medium, at least one inlet (26b, c) and one outlet (25a, b, c, d) for the fluid or the liquid (16), in particular wherein the modules are designed to be combined at will, wherein in particular in the module one locking device (31b) each for the transport line (20) and/or other locking devices (31 c - h) for the inlet (26a) for pressurizing or the transport medium and/or the inlet (26b, c) and/or the outlet (25a, b, c, d) for the fluid or the liquid (16) are provided.

13. Method for monitoring the filling level of a medium, in particular a cleaning, disinfection, or care medium in a storage container of a cleaning device for at least one dental instrument, according to one of the preceding claims, comprising the steps:
- sensor-supported recording of a static pressure in the storage container before removing medium,
- evaluation of the recorded values, and
- deriving a filling level of the medium in the storage container by means of the recorded values.

14. Method according to claim 13, **characterized in that** the evaluation is performed in an evaluating unit (30), wherein by means of the values recorded by the sensor (11), in particular by means of the values of a static pressure and/or pressure change, the amount of medium or the filling level is calculated, and/or after determining the filling level, the filling level is compared with a defined minimum value, and/or the filling level information corresponding with the filling level is transmitted to a signal.

15. Method according to one of the preceding claims 13 or 14, **characterized in that** the removed amount and/or the removal speed of the medium is determined by means of the calculated filling level and/or pressure change during or after the removal of the medium, and/or depending on the filling level the dosage device is controlled, in particular when the filling level is low the removal period of the medium from the storage container is increased.

16. Method according to one of the claims 13 to 15, **characterized in that** the shortfall of a minimum value for the filling level is determined by continuously comparing the filling level and/or pressure values calculated or recorded during removal and/or the static pressure values determined after removal with a defined suitable value for the minimum value for the filling level, and/or when the minimum value for the filling level is fallen below an optical or acoustical signal is given, and/or an outlet, associated in particular with the storage container, and/or a medium line, and/or the dosage device is locked.

## Revendications

1. Dispositif de soin destiné à au moins un appareil relevant de la technologie dentaire (4) comprenant au moins un réservoir (12) pour un fluide ou un liquide (16), en particulier un liquide de nettoyage, de désinfection ou de soin, au moins une chambre de nettoyage (40) recevant l'appareil dentaire (4) et au moins une conduite de liquide (17a, 17b) reliant le réservoir (12) et la chambre de nettoyage (40) et comportant un dispositif de mesure (10), destiné à évaluer la quantité ou le niveau du fluide ou du liquide à l'intérieur du réservoir (16), avec au moins un capteur (11) permettant de lire ces données en particulier par des techniques numériques, **caractérisé en ce que** le capteur (11) possède la configuration d'un capteur de pression.

2. Dispositif de soin selon la revendication 1, **caractérisé en ce qu'**une variation de la pression statique à l'intérieur du réservoir (12) et/ou une variation de la pression liée à un prélèvement de liquide ou à un changement du niveau de liquide peut être saisie à l'aide du capteur (11) et/ou **en ce que** le capteur de pression (11) est lié au réservoir via un tuyau de pression (29) et en particulier via un tuyau de pression (29) situé au niveau du fond (13) du réservoir (12).

3. Dispositif selon une des revendications précédentes, **caractérisé en ce qu'**une unité d'exploitation (30) destinée à l'analyse des données de pression saisies et/ou des données de variation de la pression est prévue et **en ce qu'**en particulier un indicateur optique ou sonore (32) est prévu de façon à ce que cet indicateur (32) est lié à l'unité d'exploitation (30) et/ou à une unité de contrôle (31).

4. Dispositif de soin selon une des revendications précédentes, **caractérisé en ce qu'**une saisie en continu de la pression statique est prévue ou **en ce qu'**une saisie en continu des variations de pression, en particulier pendant le prélèvement du fluide ou du liquide (16), est prévue.

5. Dispositif de soin selon une des revendications précédentes, **caractérisé en ce qu'**une unité de contrôle (31) liée à l'unité d'exploitation (30) et agissant sur le dispositif de dosage (18) est prévue pour le contrôle de la quantité prélevée et/ou de la durée de prélèvement du fluide ou du liquide (16) du réservoir (12) de façon à ce que le réglage de la quantité et/ou de la durée du prélèvement s'effectue en lien avec le niveau de remplissage et/ou de la pression statique et/ou de l'évolution des variations de pression.

6. Dispositif de soin selon une des revendications précédentes, **caractérisé en ce qu'**une unité de stockage (33) destinée à saisir et à stocker des données concernant le moment de fonctionnement, la durée de fonctionnement, le moment de prélèvement de fluide, la quantité de prélèvement de fluide, la durée de prélèvement de fluide, les pressions saisies par rapport au niveau présent dans le réservoir et/ou la quantité de prélèvement de fluide du réservoir avant et après le prélèvement de fluide, le passage en-dessous du niveau minimal, le moment de la fermeture de la sortie et/ou du dispositif de dosage et/ou de la vitesse de prélèvement.

7. Dispositif de soin selon une des revendications précédentes, **caractérisé par** :
au moins deux réservoirs pour un fluide ou un liquide, en particulier une substance de désinfection, de nettoyage ou de soin,
une conduite de fluide pour chacun des réservoirs liant le réservoir et la chambre de nettoyage, et
un dispositif de dosage et de distribution (18) pour la substance de nettoyage ou de soin situé dans les conduites (50a,b 17a-h),
de façon à ce que le dispositif de dosage et de distribution (18) possède une chambre de réception (70) commune à toutes les conduites de fluide branchées aux réservoirs et cette chambre de réception (70), en servant comme conduite de transport, fait partie de la conduite de fluide et permet sous effet de gravité de transporter le fluide (16) du réservoir (12a, 12b) vers la chambre de réception du dispositif de dosage et de distribution (18).

8. Dispositif de soin selon la revendication 7, **caractérisé en ce qu'**une introduction du fluide ou du liquide (16) dans la chambre de nettoyage (40) est prévue après l'ajout de pression, en particulier après l'application d'une surpression, d'une sous-pression ou d'une surpression d'air, l'application de pression étant en particulier prévue au niveau du dispositif de dosage et de distribution (18) avec un dispositif d'ajustement définissant l'amplitude de la pression appliquée et/ou **en ce que** le dispositif de dosage et de distribution (18) comprend au moins :
une conduite de transport (20) destinée au fluide de nettoyage ou de soin (16),
au moins un dépôt intermédiaire (33a,b) situé dans ou attribué à la conduite de transport (20) pour le fluide de soin (16) de façon à ce que attribué à cette conduite de transport (20) ou
en particulier s'ouvrant vers la conduite de transport (20) existent au moins :
une entrée (26a) pour l'application d'une surpression et/ou l'introduction d'un fluide de transport,
une entrée (26b, 26c) pour le fluide de soin (16) et au moins une sortie (25a,b,c,d) pour le fluide (16) et **en ce qu'**à l'aide de la conduite de fluide (50a,b 17a-h) pour le fluide ou le liquide (16) l'entrée (26b, 26c) est connectée au réservoir (12a, 12b) et la sortie (25a,b,c,d) est connectée à la chambre de nettoyage (40).

9. Dispositif de soin selon une des revendications précédentes 7 et 8, **caractérisé en ce qu'**au moins un premier dispositif de blocage (31a), en particulier une électrovanne et/ou un volet ou une coulisse, est prévu(e) pour la division de la conduite de transport (20) et/ou **en ce qu'**il existe un dispositif de blocage supplémentaire (31b-h), et en particulier une électrovanne supplémentaire, attribué à chacune des entrées (26a) pour l'application d'une surpression ou l'entrée d'un fluide de transport et/ou à une entrée (26b,c) et/ou à l'a sortie (25a,b,c,d) pour le fluide de soin.

10. Dispositif de soin selon une des revendications précédentes 7 à 9, **caractérisé en ce qu'**une unité d'analyse et de contrôle (30) est prévue pour le ou les dispositif (s) de blocage (31a-h) comportant en particulier au moins un programme de contrôle avec, en particulier, la possibilité de pouvoir au moins contrôler les moments d'ouverture et de fermeture et/ou la séquence et/ou les durées d'ouverture et de fermeture du ou des dispositif(s) de blocage (31a-h) et/ou **en ce que** l'actionnement du dispositif de blocage (31c,g) associé à l'entrée (26b,c) pour le fluide ou le liquide (16) s'effectue en fonction des valeurs de niveau du fluide de soin à l'intérieur du réservoir (12a,12b) et en particulier basé sur les valeurs de pression liées au niveau de remplissage du fluide.

11. Dispositif de soin selon une des revendications précédentes 8 à 10, **caractérisé en ce que** le dépôt intermédiaire (33a,b) est situé près de l'entrée (26b,c) du fluide de soin (16) et/ou **en ce que** le dépôt intermédiaire (33a,b) est situé en forme de bassin ou de canal dans la conduite de transport (20) et/ou dans une partie agrandie de l'extrémité de la conduite de transport et/ou **en ce que** la conduite de transport (20) est située dans le dispositif de dosage et de distribution (18) de préférence de façon inclinée par rapport à l'axe horizontal de sorte à ce que l'extrémité basse (22,23) de la conduite de transport (20) sert comme dépôt intermédiaire (33a,b).

12. Dispositif de soin selon une des revendications précédentes 7 à 11, **caractérisé en ce que** l'entrée (26a) pour l'application de la surpression et/ou du fluide de transport est prévue à l'intérieur du dispositif de dosage et de distribution (18) près d'une première ou d'une deuxième extrémité (22, 23) de celui-ci par rapport à l'entrée (26b,c) du fluide ou du liquide (16) ou la sortie (25a,b,c,d) du fluide ou du liquide (16) lesquelles sont situées selon la direction de transport du fluide de soin (16) après l'entrée (26a) et/ou **en ce que** le dispositif de dosage et de distribution (18) est fabriqué en au moins un module et **en ce que** ce chacun de ces modules comporte au moins une partie de conduite de transport (20), au moins un dépôt intermédiaire (33a,b), au moins une entrée (26a) permettant d'appliquer une surpression ou le fluide de transport , au moins une entrée (26b,c) et une sortie (25a,b,c,d) pour le fluide ou le liquide (16) avec en particulier la possibilité de combiner librement ces modules qui chacun possèdent en particulier un dispositif de blocage (31b) pour la conduite de transport (20) et/ou **en ce que** d'autres dispositifs de blocage (31c-h) sont prévus pour l'entrée (26a) pour l'application d'une surpression ou du fluide de transport et/ou l'entrée (26b,c) et/ou la sortie (25a,b,c,d) du fluide ou du liquide (16).

13. Procédé de contrôle du niveau de remplissage par un fluide, en particulier par un fluide de nettoyage, de désinfection ou de soin à l'intérieur d'un réservoir d'un dispositif de soin destiné au moins à un appareil relevant de la technologie dentaire selon une des revendications précédentes, comprenant les étapes suivantes :
saisie par capteur de la pression statique existante à l'intérieur d'un réservoir avant prélèvement d'un fluide, analyse des données saisies et
dérivation d'un niveau de remplissage du fluide à l'intérieur du réservoir grâce aux données saisies.

14. Procédé selon la revendication 13, **caractérisé en ce que** l'analyse est effectuée par une unité d'analyse (30) permettant de calculer la quantité de fluide ou le niveau de remplissage à partir des valeurs de pression statique et/ou de la variation de la pression saisie (s) par le capteur (11) et/ou **en ce que** le niveau de remplissage une fois déterminé est comparé à une valeur minimale prédéfinie et/ou **en ce que** l'information concernant le niveau de remplissage est envoyée sur un afficheur.

15. Procédé selon une des revendications 13 ou 14, **caractérisé en ce qu'**un calcul de la quantité prélevée et/ou de la vitesse de prélèvement du fluide est effectué basé sur le niveau de remplissage et/ou de la variation de pression calculé(e) pendant ou après le prélèvement du fluide et/ou **en ce que** le dispositif de dosage est piloté en fonction du niveau de remplissage et **en ce qu'**en particulier la durée de prélèvement du fluide du réservoir est augmentée lorsque le niveau de remplissage est bas.

16. Procédé selon une des revendications précédentes 13 à 15, **caractérisé en ce qu'**un passage en-dessous d'une valeur minimale du niveau de remplissage est détecté par une comparaison en continu des niveaux de remplissage calculés ou saisies pendant le prélèvement et/ou des pressions statiques calculées après prélèvement avec une valeur prédéfinie et correspondant à une valeur minimale du niveau de remplissage et/ou **en ce que** le passage en-dessous d'une valeur minimale du niveau de remplissage produit en particulier un signal optique ou sonore et/ou un blocage en particulier d'une sortie liée au réservoir et/ou d'une conduite de fluide et/ou du dispositif de dosage.
